# EUROPÄISCHE PATENTSCHRIFT

(11) **EP 1 417 186 B1**
(45) Veröffentlichungstag und Bekanntmachung des Hinweises auf die Patenterteilung: **16.03.2011**
(21) Anmeldenummer: 02751158.3
(22) Anmeldetag: 24.07.2002
(51) Int. Cl.: C07D 271/06, A61K 31/42, A61P 31/18

(54) **HETEROCYCLYLARYLSULFONAMIDE**
HETEROCYCLIC ARYL SULPHONAMIDES
HETEROCYCLYLARYLSULFONAMIDES

(30) Priorität: 06.08.2001 DE 10138578
(43) Veröffentlichungstag der Anmeldung: 12.05.2004
(73) Patentinhaber: AiCuris GmbH & Co. KG, 42117 Wuppertal (DE)
(72) Erfinder: WUNBERG, Tobias, 42699 Solingen (DE); BENDER, Wolfgang, 42113 Wuppertal (DE); ECKENBERG, Peter, 42115 Wuppertal (DE); HALLENBERGER, Sabine, 40219 Düsseldorf (DE); HENNINGER, Kerstin, 42115 Wuppertal (DE); KELDENICH, Jörg, 42113 Wuppertal (DE); KERN, Armin, 42109 Wuppertal (DE); RADDATZ, Siegfried, 51065 Köln (DE); REEFSCHLÄGER, Jürgen, 26125 Oldenburg (DE); SCHMIDT, Gunter, 42115 Wuppertal (DE); ZIMMERMANN, Holger, 42113 Wuppertal (DE); ZUMPE, Franz, 42109 Wuppertal (DE); RADTKE, Martin, 40699 Erkrath (DE)
(74) Vertreter: Witte, Weller & Partner
(86) Internationale Anmeldenummer: PCT/EP2002/008243
(87) Internationale Veröffentlichungsnummer: WO 2003/014094

(56) Entgegenhaltungen:
- WO-A-01/02350
- WO-A-99/37291

## Beschreibung

Die vorliegende Erfindung betrifft neue Verbindungen, Verfahren zu ihrer Herstellung und ihre Verwendung als Arzneimittel, insbesondere als antivirale Mittel, insbesondere gegen Cytomegalieviren.

Die Verbindung 2,2-Dimethyl-N-[4-[[[4-(4-phenyl-2H-1,2,3-triazol-2-yl)phenyl]-sulfonyl]amino]phenyl]-propanamid ist als antiviral wirksam bekannt aus WO 99/37291.

Eine Aufgabe der vorliegenden Erfindung ist es, alternative oder besser wirksame Mittel gegen Cytomegalieviren bereitzustellen.

Die vorliegende Erfindung betrifft Verbindungen der allgemeinen Formel (I) 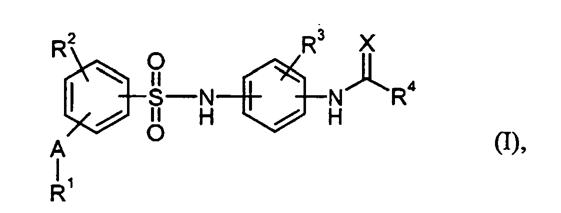 in der
R² und R³ gleich oder verschieden sind und für Wasserstoff, Hydroxy, Halogen, Nitro, Cyano, Trifluormethyl, Trifluormethoxy, (C₁-C₆)-Alkyl, (C₁-C₆)-Alkoxy oder für eine Gruppe der Formel 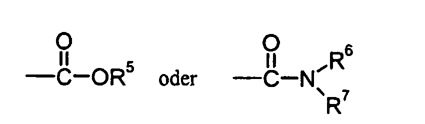 stehen,
in denen
R⁵, R⁶ und R⁷ gleich oder verschieden sind und jeweils für Wasserstoff oder (C₁-C₆)-Alkyl stehen, das seinerseits substituiert sein kann mit ein oder zwei Substituenten, ausgewählt aus der Gruppe bestehend aus Hydroxy, Halogen, Cyano, Trifluormethyl und Trifluormethoxy,
- A: für über ein C-Atom mit dem benachbarten Phenylring verknüpftes fünf- oder sechsgliedriges Heteroaryl mit bis zu drei Heteroatomen aus der Reihe S, N und/oder O steht

- R¹: für den Rest 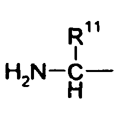
steht,
worin
- R¹¹: für die Seitengruppe einer Aminosäure steht, und die Aminogruppe in R¹ gegebenenfalls ein- oder mehrfach substituiert sein kann mit (C₁- C₆)Alkyl, Alkylcarbonyl, Phenyl,
oder
- R¹: für einen geradkettigen oder verzweigten (C₁-C₅)-Alkylrest steht, der seinerseits substituiert sein kann mit einer oder mehreren Gruppen ausgewählt aus Phe- nyl, Piperidinyl, Pyridinyl, Thiazolyl, Thienyl, 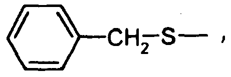 einer Gruppe 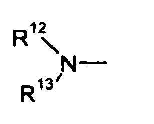
worin
R¹² und R¹³ gleich oder verschieden sind und stehen können für Wasserstoff, (C₁-C₆)Alkyl, Alkylcarbonyl, eine Aminoschutzgruppe, Phenyl,
oder
- R¹: für einen Rest steht, oder
- R¹: für einen geradkettigen oder verzweigten (C₁-C₅)-Alkylrest steht, der seinerseits substituiert ist mit einer Gruppe 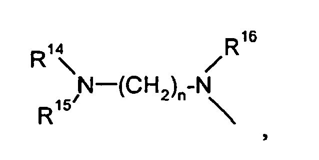
worin
R¹⁴, R¹⁵, R¹⁶ gleich oder verschieden sind und für Wasserstoff oder (C₁-C₆)Akyl stehen
und
- n: die Werte 2 oder 3 annehmen kann,
oder
- R¹: für Piperidinyl oder den Rest R13 steht, worin R¹² und R¹³ die oben angegebene Bedeutung haben,
- n: für eine Zahl von 1 bis 4 steht und der Ring bis zu dreifach gleich oder verschieden mit Halogen, (C₁-C₆)-Alkyl, Halogen-(C₁-C₆)-Alkyl, Amino, Hydroxy substituiert sein kann,
- R⁴: für tert.-Butyl, das gegebenenfalls bis zu dreifach, gleich oder verschieden, durch Hydroxy, Fluor oder Chlor substituiert ist, oder für Cyclopropyl oder Cyclobutyl steht, die ein- bis dreifach gleich oder unabhängig durch Halgoen oder (C₁-C₆)-Alkyl substituiert sind, woei (C₁- C₆)-Alkyl gegebenenfalls durch Hydroxy, Fluor oder Chlor substituiert ist,
und in der
- X: für Sauerstoff oder Schwefel steht,
und worin stickstoffhaltige Heterocyclen auch als N-Oxide vorliegen können,
sowie deren Tautomere, Stereoisomere, stereoisomere Gemische und deren pharmakologisch verträgliche Salze.

(C₁-C₆)-Alkyl steht im Rahmen der Erfindung für einen geradkettigen oder verzweigten Alkylrest mit 1 bis 6 Kohlenstoffatomen. Beispielsweise seien genannt: Methyl, Ethyl, n-Propyl, Isopropyl, n-Butyl, t-Butyl, n-Pentyl und n-Hexyl.

(C₃-C₆)-Cycloalkyl steht im Rahmen der Erfindung für eine Cycloalkylgruppe mit 3 bis 6 Kohlenstoffatomen. Beispielsweise seien genannt: Cyclopropyl, Cyclobutyl, Cyclopentyl und Cyclohexyl.

(C₁-C₆)-Alkoxy steht im Rahmen der Erfindung für einen geradkettigen oder verzweigten Alkoxyrest mit 1 bis 6 Kohlenstoffatomen. Beispielsweise seien genannt: Methoxy, Ethoxy, n-Propoxy, Isopropoxy, t-Butoxy, n-Pentoxy und n-Hexoxy. Bevorzugt sind Methoxy und Ethoxy.

(C₆-C₁₀)-Aryl steht im Rahmen der Erfindung für einen aromatischen Rest mit 6 bis 10 Kohlenstoffatomen. Bevorzugte Arylreste sind Phenyl und Naphthyl.

Aralkyl steht im Rahmen der Erfindung für (C₆-C₁₀)-Aryl, das seinerseits an (C₁-C₄)Alkyl gebunden ist. Bevorzugt ist Benzyl.

Mono-(C₁-C₆)-Alkylamino steht im Rahmen der Erfindung für eine Amino-Gruppe mit einem geradkettigen, verzweigten oder cyclischen Alkylsubstituenten, der 1 bis 6 Kohlenstoffatome aufweist. Beispielsweise seien genannt: Methylamino, Ethylamino, n-Propylamino, Isopropylamino, Cyclopropylamino, t-Butylamino, n-Pentylamino, Cyclopentylamino und n-Hexylamino.

Di-(C₁-C₆)-Alkylamino steht im Rahmen der Erfindung für eine Amino-Gruppe mit zwei gleichen oder verschiedenen geradkettigen, verzweigten oder cyclischen Alkylsubstituenten, die jeweils 1 bis 6 Kohlenstoffatome aufweisen. Beispielsweise seien genannt: *N,N-*Dimethylamino, *N,N-*Diethylamino, *N-*Ethyl-*N*-methylamino, *N-*Methyl-*N-*n-propylamino, *N-*Methyl-*N*-cyclopropylamino, *N-*Isopropyl-*N-*n-propylamino, *N-*t-Butyl-*N-*methylamino, *N-*Ethyl-*N-*n-pentylamino und *N-*n-Hexyl-*N-*methylamino.

Heteroaryl steht im Rahmen der Erfindung für einen monocyclischen Heteroaromaten mit bis zu 3 Heteroatomen aus der Reihe S, N und/oder O, der über ein Ringkohlenstoffatom des Heteroaromaten verknüpft ist. Beispielsweise seien genannt: Furan-2-yl, Furan-3-yl, Pyrrol-1-yl, Pyrrol-2-yl, Pyrrol-3-yl, Thienyl, Thiazolyl, Oxazolyl, Imidazolyl, Triazolyl, Pyridyl, Pyrimidyl, Pyridazinyl. Bevorzugt sind Oxadiazolyl, Thiadiazolyl.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

Ein 3- bzw. 5-verknüpftes 1,2,4-Oxadiazol steht für ein Oxadiazol, das über das 3- bzw. 5-Ringkohlenstoffatom an das Phenylsulfonamid gebunden ist.

Unter der Seitengruppe einer Aminosäure wird im Rahmen der Erfindung beispielsweise Wasserstoff (Glycin), Methyl (Alanin), Propan-2-yl (Valin), 2-Methyl-propan-1-yl (Leucin), 1-Methyl-propan-1-yl (Isoleucin), eine Propan-1,3-diyl-Gruppe, die mit dem Stickstoffatom der Aminogruppe verbunden ist (Prolin), eine 2-Hydroxypropan-1,3-diyl-Gruppe, die mit dem Stickstoffatom der Aminogruppe verbunden ist (Hydroxyprolin), eine Gruppe der Formel 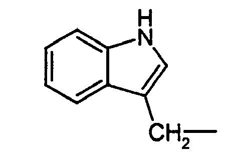 (Tryptophan), eine Benzylgruppe (Phenylalanin), eine Methylthioethylgruppe (Methionin), Hydroxymethyl (Serin), p-Hydroxybenzyl (Tyrosin), 1-Hydroxy-ethan-1-yl (Threonin), Mercaptomethyl (Cystein), Carbamoylmethyl (Asparagin), Carbamoylethyl (Glutamin), Carboxymethyl (Asparaginsäure), Carboxyethyl (Glutaminsäure), 4-Aminobutan-1-yl (Lysin), 3-Guanidinopropan-1-yl (Arginin), Imidazol-4-ylmethyl (Histidin), 3-Ureidopropan-1-yl (Citrullin), Mercaptoethyl (Homocystein), Hydroxyethyl (Homoserin), 4-Amino-3-hydroxybutan-1-yl (Hydroxylysin), 3-Amino-propan-1-yl (Ornithin), verstanden.

Aminoschutzgruppe steht im Rahmen der vorliegenden Erfindung für eine Schutzgruppe, die die Aminogruppe unempfindlich macht gegenüber einigen Reaktionsbedingungen, die sich aber unter anderen Reaktionsbedingungen einfach wieder entfernen lässt, siehe T. W. Greene, P. G. Wuts, Protective Groups in Organic Synthesis, 3rd ed., John Wiley, New York, 1999. Bevorzugte Aminoschutzgruppen sind Carbamate, z.B. *tert*-Butyloxycarbonyl (Boc), 9-Fluorenylmethyloxycarbonyl (FMOC) oder Benzyloxycarbonyl (Cbz- / Z-) oder andere Oxycarbonylderivative.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen können Säureadditionssalze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin, oder abgeleitet von natürlichen Aminosäuren wie beispielsweise Glycin, Lysin, Arginin oder Histidin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

Außerdem umfaßt die Erfindung auch Prodrugs der erfindungsgemäßen Verbindungen. Als "Prodrugs" werden erfindungsgemäß solche Derivate der Verbindungen der allgemeinen Formel (I) bezeichnet, welche selbst biologisch weniger aktiv oder auch inaktiv sein können, jedoch nach Applikation unter physiologischen Bedingungen in die entsprechende biologisch aktive Form überführt werden (beispielsweise metabolisch, solvolytisch oder auf andere Weise).

Die oben aufgeführten allgemeinen oder in Vorzugsbereichen angegebenen Rests-Definitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen angegebenen Restedefinitionen werden unabhängig von den jeweilig angegebenen Kombinationen der Reste beliebig auch durch Restedefinitionen anderer Kombinationen ersetzt.

Die Erfindung betrifft bevorzugt Verbindungen der allgemeinen Formel (I),
in der
R² und R³ gleich oder verschieden sind und für Wasserstoff oder Halogen stehen,
- A: für den Rest (A-I) 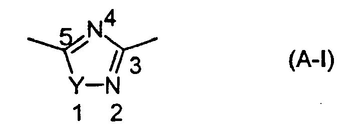
steht, der über eines der Kohlenstoffatome der Positionen 3 oder 5 mit dem benachbarten Phenylring verbunden ist,
und in dem
- Y: für Sauerstoff steht,
oder
- A: für den Rest (A-II) 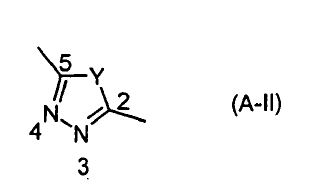
steht, der über eines der Kohlenstoffatome der Positionen 2 oder 5 mit dem benachbarten Phenylring verbunden ist,
und in dem
- Y: für Sauerstoff steht,

- R¹: für den Rest 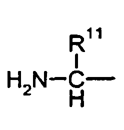 steht,
worin
- R¹¹: für die Seitengruppe einer Aminosäure steht, und die Aminogruppe in R¹ gegebenenfalls ein- oder mehrfach substituiert sein kann mit (C₁- C₆)-Alkyl, Alkylcarbonyl, einer Aminoschutzgruppe, Phenyl,
oder
- R¹: für einen geradkettigen oder verzweigten (C₁C₅)-Alkylrest steht, der seinerseits substituiert sein kann mit einer oder mehreren Gruppen ausgewählt aus Phenyl, Piperidinyl, Pyridinyl, Thiazolyl, Thienyl, 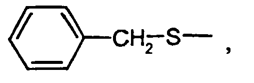 einer Gruppe 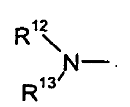
worin
R¹² und R¹³ gleich oder verschieden sind und stehen können für Wasserstoff, (C₁-C₆)-Alkyl, Alkylcarbonyl, eine Aminoschutzgruppe, Phenyl,
oder
- R¹: für einen geradkettigen oder verzweigten (C₁-C₅)-Alkylrest steht, der seinerseits substituiert ist mit einer Gruppe 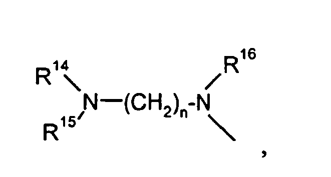
worin
R¹⁴, R¹⁵, R¹⁶ gleich oder verschieden sind und für Wasserstoff oder Methyl stehen
und
- n: die Werte 2 oder 3 annehmen kann,
oder
- R¹: für Piperidin-3-yl oder den Rest 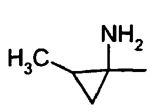 steht,

- R⁴: für tert.-Butyl, das gegebenenfalls bis zu dreifach, gleich oder verschieden, durch Hydroxy, Fluor oder Chlor substituiert ist, oder
für Cyclopropyl oder Cyclobutyl steht, das α-ständig zur Carbonylgruppe oder Thiocarbonylgruppe durch Methyl substituiert ist, welches seinerseits gegebenenfalls durch Hydroxy, Fluor oder Chlor substituiert ist,
und in der
- X: für Sauerstoff steht,
und worin stickstoffhaltige Heterocyclen auch als N-Oxide vorliegen können,
sowie deren Tautomere, Stereoisomere, stereoisomere Gemische und deren pharmakologisch verträglichen Salze.

Die Erfindung betrifft besonders bevorzugt Verbindungen der allgemeinen Formel (I),
in der
R² und R³ für Wasserstoff stehen,
- A: für einen der Reste 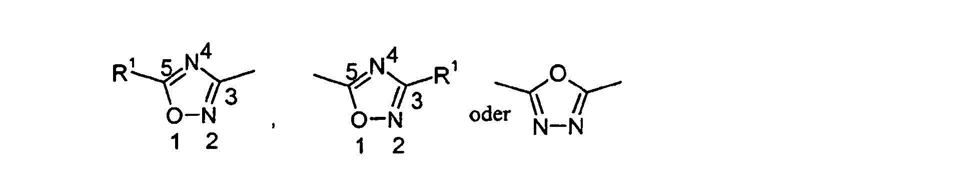
steht,
- R¹: für den Rest 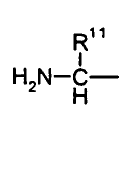 steht,
worin
- R¹¹: für die Seitengruppe einer Aminosäure steht, und die Aminogruppe in R¹ gegebenenfalls ein- oder mehrfach substituiert sein kann mit Methyl, Alkylcarbonyl, einer Aminoschutzgruppe, Phenyl,
oder
- R¹: für einen geradkettigen oder verzweigten (C₁-C₅)-Alkylrest steht, der seinerseits substituiert sein kann mit einer oder mehreren Gruppen ausgewählt aus Phenyl, Piperidinyl, Pyridinyl, Thiazolyl, Thienyl, 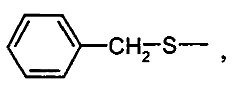 einer Gruppe 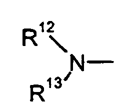
worin
R¹² und R¹³ gleich oder verschieden sind und stehen können für Wasserstoff, Methyl, Alkylcarbonyl, eine Aminoschutzgruppe, Phenyl,
oder
- R¹: für einen geradkettigen oder verzweigten (C₁-C₅) Alkylrest steht, der seinerseits substituiert ist mit einer Gruppe 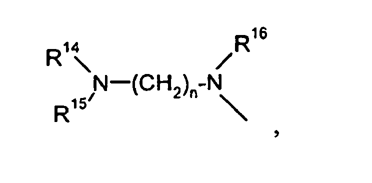
worin
R¹⁴, R¹⁵, R¹⁶ gleich oder verschieden sind und für Wasserstoff oder Methyl stehen
und
- n: die Werte 2 oder 3 annehmen kann,
oder
- R¹: für Piperidin-3-yl oder den Rest 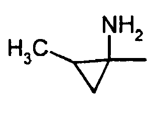 steht,
- R⁴: für tert.-Butyl, das gegebenenfalls bis zu dreifach, gleich oder verschieden, durch Hydroxy, Fluor oder Chlor substituiert ist, oder für Cyclopropyl oder Cyclobutyl steht, das α-ständig zur Carbonylgruppe oder Thiocarbonylgruppe durch Methyl substituiert ist, welches seinerseits gegebenenfalls durch Hydroxy, Fluor oder Chlor substituiert ist,
und in der
- X: für Sauerstoff steht,
und worin stickstoffhaltige Heterocyclen auch als N-Oxide vorliegen können,
sowie deren Tautomere, Stereoisomere, stereoisomere Gemische und deren pharmakologisch verträglichen Salze.

In einer bevorzugten Ausführungsform betrifft die Erfindung Verbindungen der allgemeinen Formel (Ia) 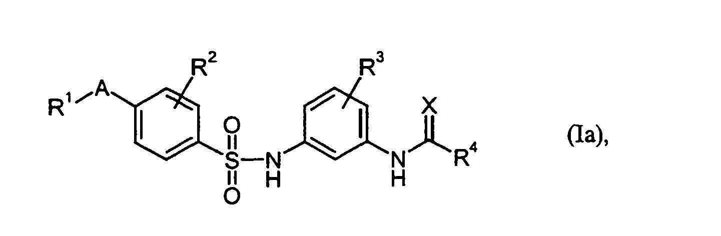 in der
R¹, R², R³, R⁴, A und X die oben angegebenen Bedeutungen haben.

In einer weiteren bevorzugten Ausführungsform betrifft die Erfindung solche Verbindungen der allgemeinen Formel (I),
in denen
- R⁴: für einen der Reste 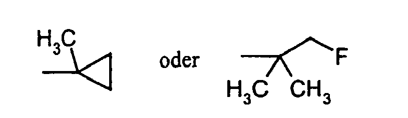
steht.

In einer weiteren bevorzugten Ausführungsform betrifft die Erfindung solche Verbindungen der allgemeinen Formel (I),
in denen
- A: für ein 3-verknüpftes 1,2,4-Oxadiazol steht.

Ganz besonders bevorzugte Verbindungen der vorliegenden Erfindung sind Sulfonamide, die ausgewählt sind aus der Gruppe der folgenden Verbindungen:

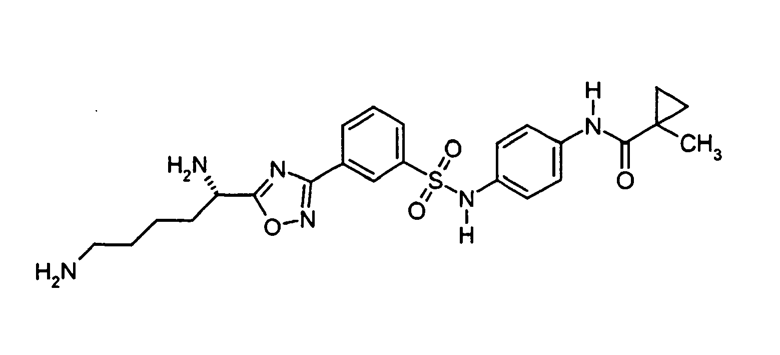

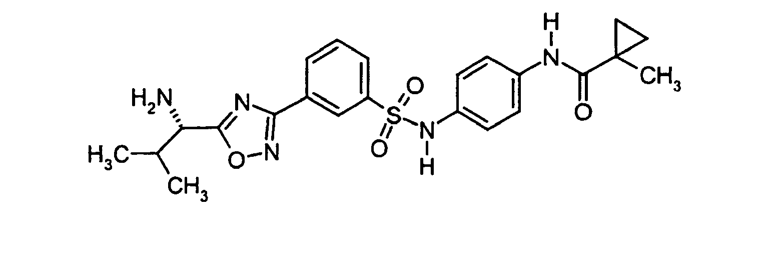

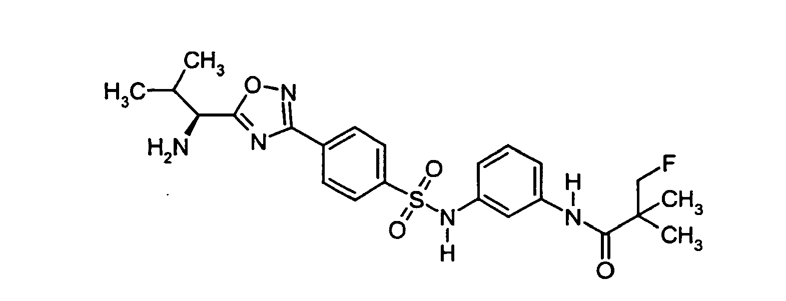

Die Erfindung betrifft weiter Verfahren zur Herstellung von Verbindungen der allgemeinen Formel (I), dadurch gekennzeichnet, dass man

### [A] Nitro-Aniline der allgemeinen Formel [A-1]

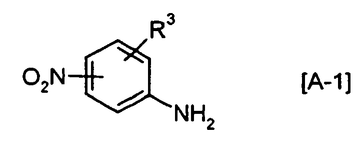

worin
- R³: die oben angegebene Bedeutung hat,
mit Verbindungen der allgemeinen Formel [A-2] 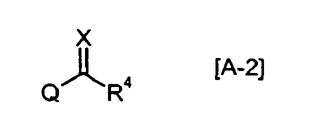 worin
X und R⁴ eine der oben angegebenen Bedeutungen haben,
und
- Q: für eine Abgangsgruppe, z. B. Halogen, vorzugsweise für Chlor oder Brom steht,
in inerten Lösemitteln in Gegenwart einer Base zu Verbindungen der allgemeinen Formel [A-3] 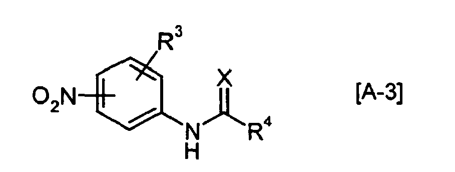 worin
X, R³ und R⁴ eine der oben angegebenen Bedeutungen haben,
umsetzt,
und

### [B] die Nitro-Aromaten der allgemeinen Formel [A-3], beispielsweise in Gegenwart von Übergangsmetallkatalysatoren und Wasserstoff, in inerten Lösemitteln zu aromatischen Aminen der allgemeinen Formel [B-1]

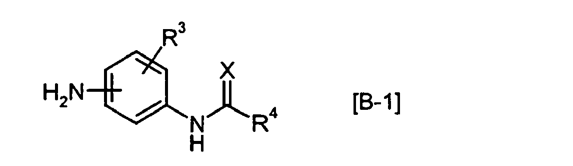

worin
X, R³ und R⁴ eine der oben angegebenen Bedeutungen haben,
reduziert,
und

### [C] Amine der allgemeinen Formel [B-1] mit Sulfonsäurederivaten der allgemeinen Formel [C-1]

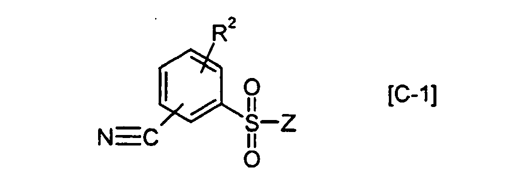

worin
- R²: die oben angegebene Bedeutung hat,
und
- Z: für eine Abgangsgruppe, z. B. Halogen, vorzugsweise für Chlor oder Brom steht,

in inerten Lösemitteln, in Gegenwart einer Base, zu Verbindungen der allgemeinen Fomel [C-2] 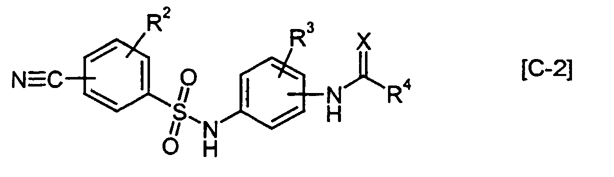 worin
X, R², R³ und R⁴ eine der oben angegebenen Bedeutungen haben,
umsetzt,
und

### [D] die Nitrile der allgemeinen Formel [C-2] in polaren protischen Lösemitteln, beispielsweise Alkoholen, bei erhöhter Temperatur, vorzugsweise der Siedetemperatur des Lösemittels, in Gegenwart einer Base mit Hydroxylamin zu Amidoximen der allgemeinen Formel [D-1]

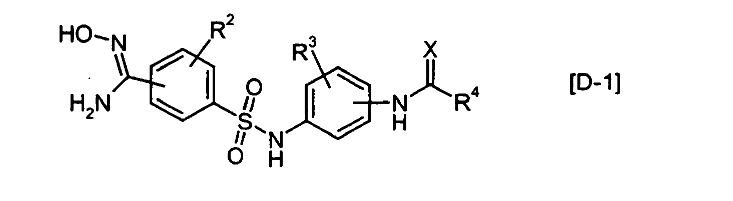

worin
X, R², R³ und R⁴ eine der oben angegebenen Bedeutungen haben,
umsetzt,
und

### [E] Amidoxime der allgemeinen Formel [D-1] mit einer Carbonsäure der allgemeinen Formel [E-1]

R¹-COOH [E-1]

worin
- R¹: die oben angegebene Bedeutung hat und in R¹ enthaltene Amino-Gruppen mit aus der Peptidchemie bekannten Schutzgruppen, wie beispielsweise der Boc- Schutzgruppe, geschützt vorliegen,
in Gegenwart eines Kondensationsmittels, beispielsweise Benzotriazolyl-N-Oxitris(dimethylamino)phosphonium-Hexafluorophosphat (PyBOP), oder anderen aus der Peptidchemie bekannten Aktivierungsreagenzien sowie Säurechloriden, und einer Base in einem polaren, aprotischen Lösemittel, beispielsweise Tetrahydrofuran, acyliert, das acylierte Amidoxim als Rohprodukt isoliert und nachfolgend in einem hochsiedenden, polaren Lösemittel, beispielweise DMF, bei erhöhter Temperatur zum 1,2,4-Oxadiazol cyclisiert.

Das erfindungsgemäße Verfahren zur Herstellung von über die Position 3 verknüpften 1,2,4-Oxadiazolen wird durch das folgende Formelschema beispielhaft erläutert:

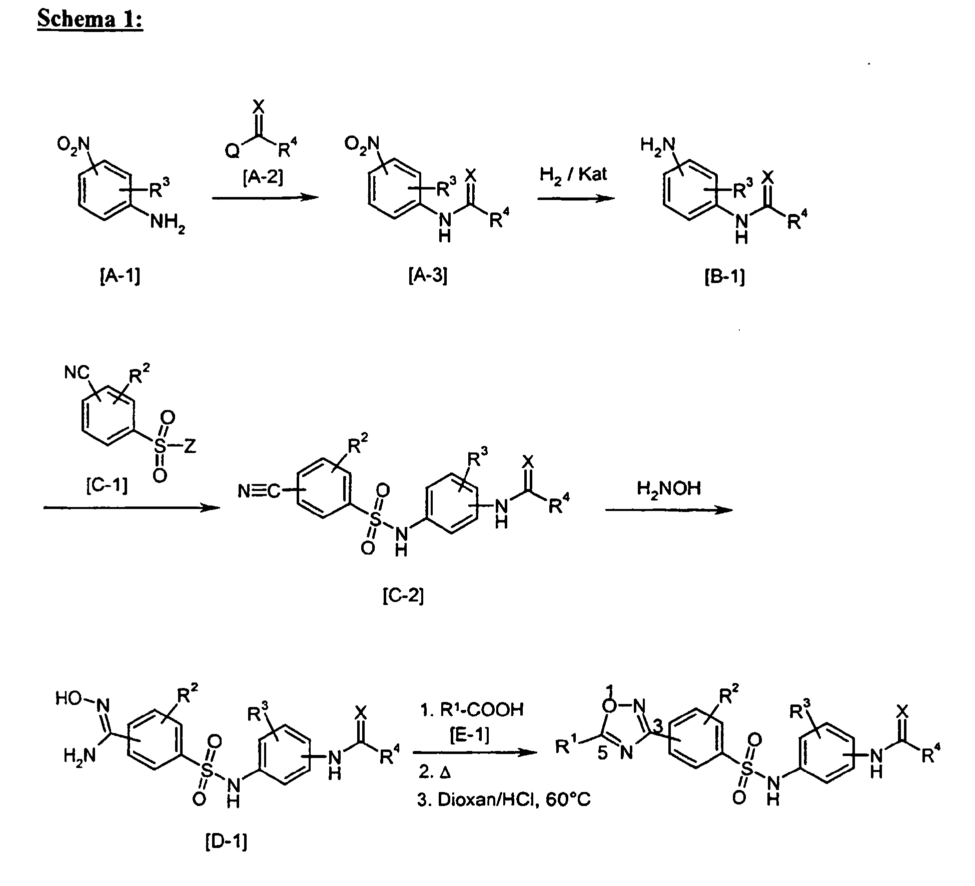

Die Erfindung betrifft weiter Verfahren zur Herstellung von Verbindungen der allgemeinen Formel (I), dadurch gekennzeichnet, dass man

### [F] Sulfonylhalogenide der allgemeinen Formel [F-1]

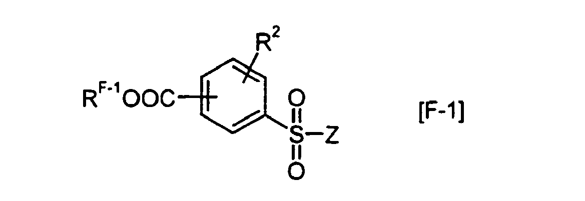

worin
R² und Z die oben angegebene Bedeutung haben,
und
- R^{F-1}: für (C₁-C₄)-Alkyl, Aralkyl oder eine Carbonsäureschutzgruppe steht,
in Gegenwart einer Base mit Anilinen der allgemeinen Formel [B-1] zu Sulfonamiden der allgemeinen Formel [F-2] 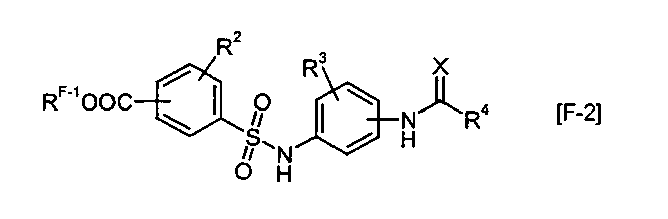 worin
R^{F-1}, R², R³, R⁴ und X die oben angegebene Bedeutung haben,
umsetzt,
und nachfolgend aus den Verbindungen der allgemeinen Formel [F-2] die Gruppe R^{F-1} beispielsweise in Gegenwart von Hydroxyl-Anionen abspaltet und zu Sulfonamiden der allgemeinen Formel [F-3] umsetzt, 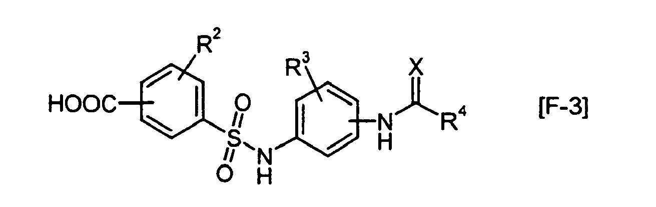 und

### [G] Amid-Oxime der allgemeinen Formel [G-1]

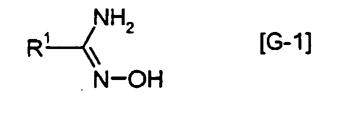

worin
- R¹: die oben angegebene Bedeutung hat,
mit Verbindungen der allgemeinen Formel [F-3] zu Verbindungen der allgemeinen Formel [G-2] kondensiert, 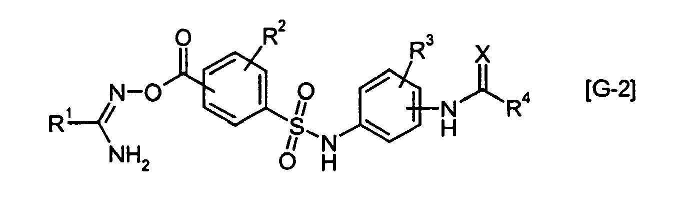 worin
R¹, R², R³, R⁴ und X die oben angegebene Bedeutung haben,
und

### [H] Verbindungen der allgemeinen Formel [G-2] thermisch zu den erfindungsgemäßen 5-verknüpften 1,2,4-Oxadiazolen der allgemeinen Formel [H-1]

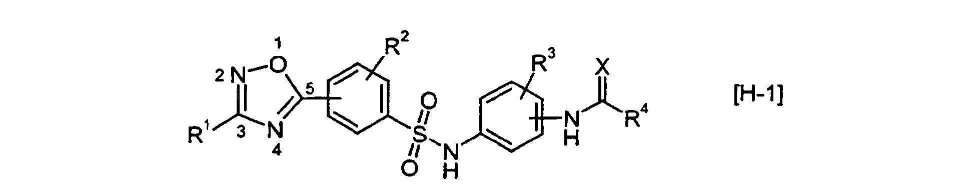

worin
R¹, R², R³, R⁴ und X die oben angegebene Bedeutung haben,
cyclisiert.

Das erfindungsgemäße Verfahren wird durch die folgenden Formelschemata beispielhaft erläutert: 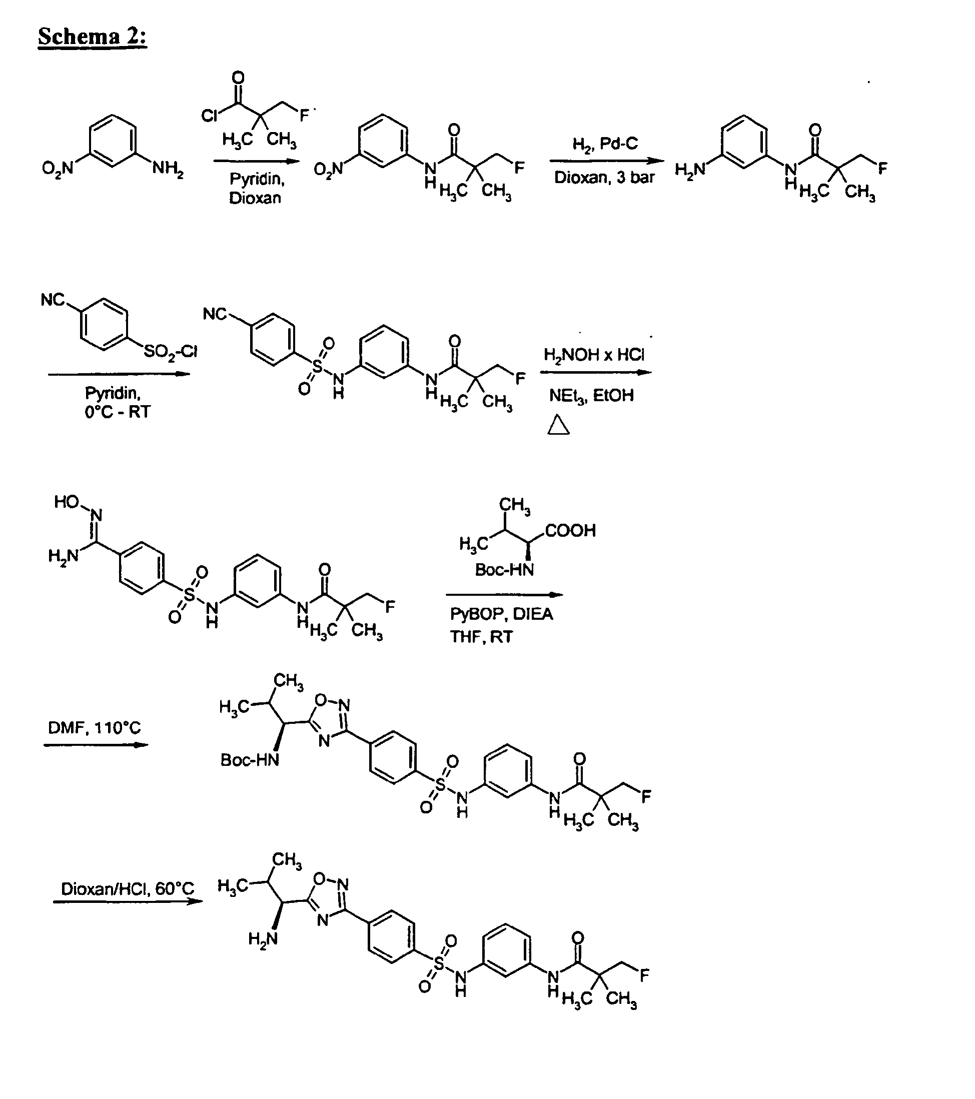 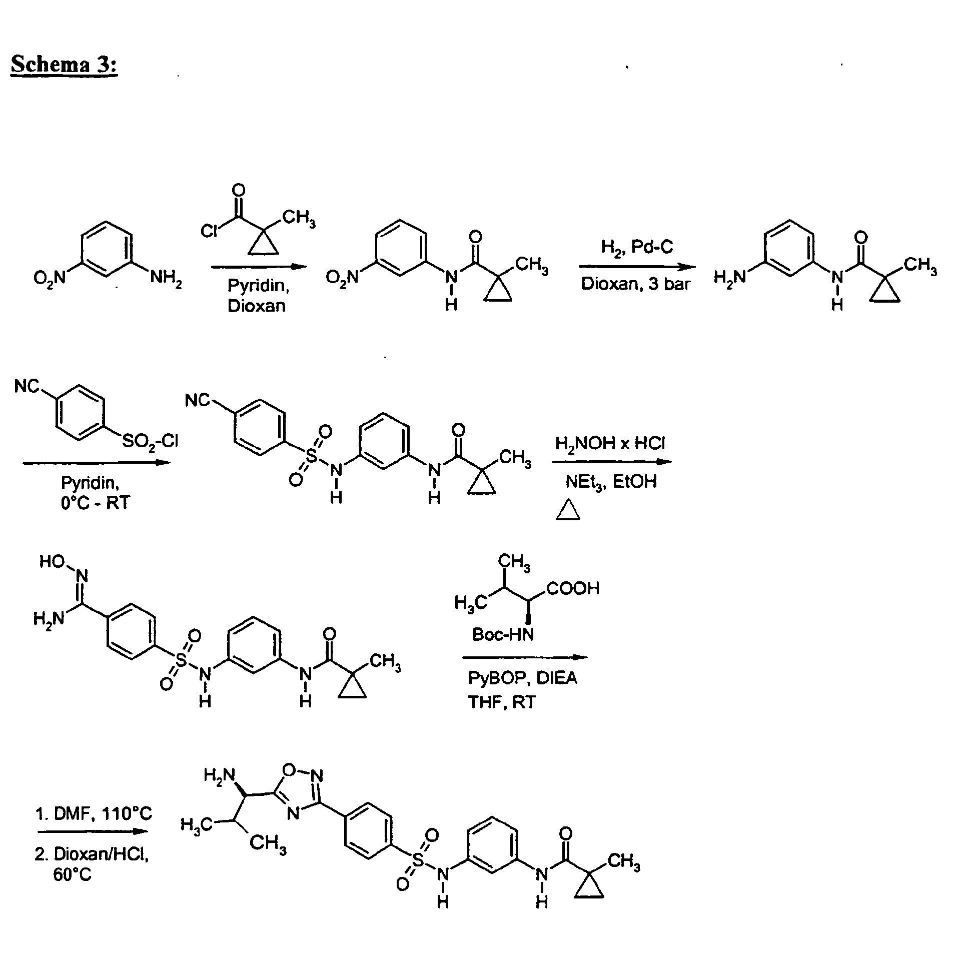 Als Lösemittel eignen sich für alle Verfahrensschritte die üblichen inerten Lösemittel, die sich unter den Reaktionsbedingungen nicht verändern. Hierzu gehören bevorzugt organische Lösemittel wie Ether, z.B. Diethylether, Glykolmono- oder -dimethylether, Dioxan oder Tetrahydrofuran, oder Alkohole wie Methanol, Ethanol, n-Propanol, iso-Propanol, n-Butanol oder tert.-Butanol, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel, gegebenenfalls auch mit Wasser zu verwenden. Besonders bevorzugt sind Methylenchlorid, Tetrahydrofuran, Dioxan und Dioxan/Wasser und insbesondere die im Textabschnitt "Allgemeine Arbeitsvorschriften" erwähnten Lösemittel.

Als Basen eignen sich organische Amine wie Tri-(C₁-C₆)-alkylamine, beispielsweise Triethylamin, oder Heterocyclen wie Pyridin, Methylpiperidin, Piperidin oder N-Methylmorpholin. Bevorzugt sind Triethylamin und Pyridin.

Die Basen werden im allgemeinen in einer Menge von 0,1 mol bis 5 mol, bevorzugt von 1 mol bis 3 mol jeweils bezogen auf 1 mol der Verbindungen der allgemeinen Formeln [A-1], [B-1], [C-2], [D-1] und [E-1] eingesetzt.

Als Carbonsäureschutzgruppe eignen sich solche, die die Carbonsäuregruppe unempfindlich machen gegenüber bestimmten Reaktionsbedingungen, die sich aber unter anderen Reaktionsbedingungen einfach wieder entfernen lässt, siehe T. W. Greene, P. G. Wuts, Protective Groups in Organic Synthesis, 3rd ed., John Wiley, New York, 1999. Bevorzugte Carbonsäureschutzgruppen sind Ester wie Alkylester oder Aralkylester, insbesondere Benzylester und -derivate.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Reaktionen werden in einem Temperaturbereich von 0°C bis 150°C, vorzugsweise bei 0°C bis 30°C und bei Normaldruck durchgeführt. Die Umsetzung der Verbindungen [G-2] zu [H-1] erfolgt bei erhöhter Temperatur, vorzugsweise bei Temperaturen oberhalb von 100°C.

Die Reduktionen können im allgemeinen durch Wasserstoff in inerten organischen Lösemitteln wie Dimethylformamid, Alkoholen, Ethern oder Essigsäureestern, oder deren Gemischen, mit Katalysatoren wie Raney-Nickel, Palladium, Palladium auf Kohle oder Platin, oder mit Hydriden oder Boranen, oder mit anorganischen Reduktionsmitteln wie beispielsweise Zinn(II)chlorid, in inerten Lösemitteln, gegebenenfalls in Anwesenheit eines Katalysators durchgeführt werden. Bevorzugt ist Palladium auf Kohle.

Die Umsetzung kann bei normalem oder bei erhöhtem Druck durchgeführt werden (z.B. 1 bis 5 bar). Im allgemeinen arbeitet man bei Normaldruck. Hydrierungen werden bevorzugt unter erhöhtem Druck, im allgemeinen bei 3 bar, durchgeführt.

Die Reduktionen werden im allgemeinen in einem Temperaturbereich von 0°C bis +60°C, vorzugsweise bei +10°C bis +40°C durchgeführt.

Als Lösemittel für die Acylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylformamid, Acetonitril oder Aceton. Ebenso ist es möglich, Gemische der genannten Lösemittel zu verwenden. Bevorzugt sind Dichlormethan, Tetrahydrofuran und Pyridin.

Die Acylierung wird in den oben aufgeführten Lösemitteln bei Temperaturen von 0°C bis +150°C, vorzugsweise bei Raumtemperatur bis +100°C und bei Normaldruck durchgeführt.

Die Verbindungen der allgemeinen Formeln [A-1], [A-2], [C-1], [E-1], [F-1] und [G-1] sind an sich bekannt oder nach literaturbekannten Methoden herstellbar. Weitere Verbindungen der allgemeinen Formel (I), in denen A für ein 1,3,4-Oxadiazol steht, können beispielsweise wie nachfolgend gemäß dem Schema 4 angeführt an einem polymeren Träger, z.B Formylharz (Fa. Nova), 0,78 mmol/g, nachfolgend "Formyl-Harz" genannt, mit dem IRORI-System nach der "Split & Mix"-Methode hergestellt werden: 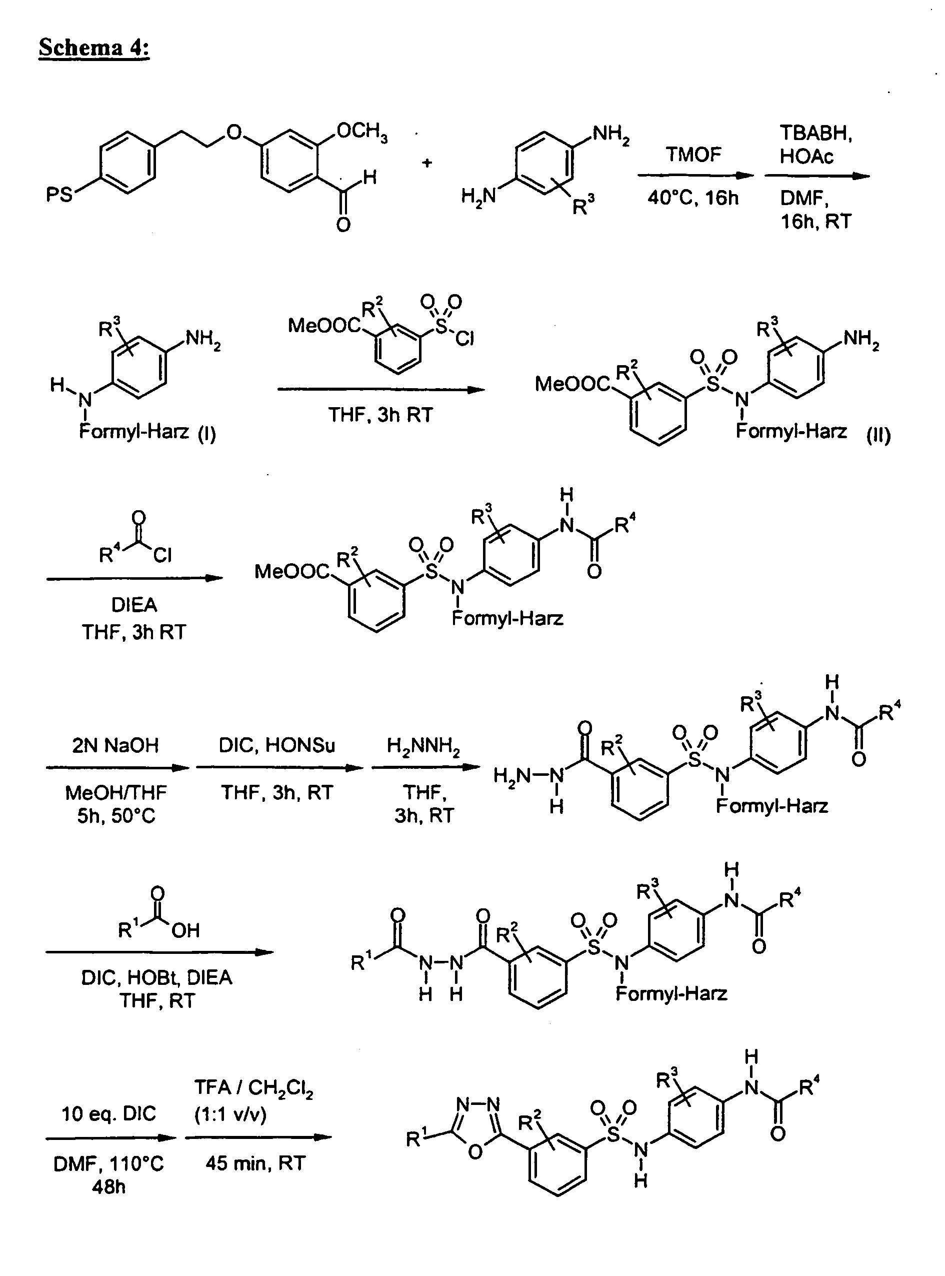 Die gemäß Schema 4 aufgezeigten Verfahren erlauben also die Herstellung weiterer erfindungsgemäßer Verbindungen der allgemeinen Formel (I), worin
- X: für Sauerstoff
und
- A: für den Rest (A-II) 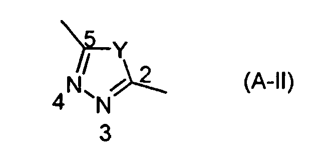 steht, der über eines der Kohlenstoffatome der Positionen 2 oder 5 mit dem benachbarten Phenylring verbunden ist,
und in dem
- Y: für Sauerstoff steht,
indem Hydrazide der allgemeinen Formel [H-2] 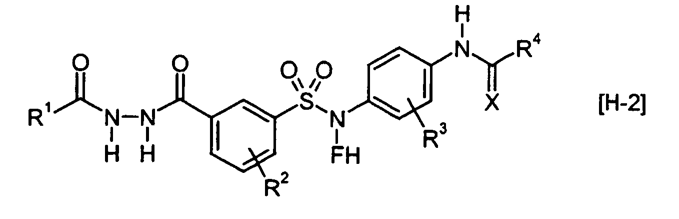 worin X, R¹, R², R³, R⁴ eine der oben angegebenen Bedeutungen haben,
und
- FH: für Wasserstoff, eine Amino-Schutzgruppe oder einen polymeren Träger steht,
unter Wasserabspaltung zu den Verbindungen der allgemeinen Formel (I) cyclisiert werden.

Die erfindungsgemäßen Verbindungen der allgemeinen Formel (I) zeigen ein nicht vorhersehbares überraschendes Wirkspektrum. Sie zeigen eine antivirale Wirkung gegenüber Vertretern der Gruppe der Herpes viridae, besonders gegenüber dem humanen Cytomegalievirus (HCMV). Sie sind somit zur Behandlung und Prophylaxe von Erkrankungen, die durch Herpes viridae, insbesondere von Erkrankungen, die durch humane Cytomegalieviren hervorgerufen werden, geeignet.

Die Verbindungen der allgemeinen Formel (I) können aufgrund ihrer besonderen Eigenschaften zur Herstellung von Arzneimitteln, die zur Prophylaxe oder Behandlung von Krankheiten, insbesondere viraler Erkrankungen, geeignet sind, verwendet werden.

Die erfindungsgemäßen Verbindungen stellen aufgrund ihrer Eigenschaften wertvolle Wirkstoffe zur Behandlung und Prophylaxe von humanen Cytomegalievirus-Infektionen und dadurch hervorgerufenen Erkrankungen dar. Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und Krebs-Therapie.

Die neuen Wirkstoffe können alleine und bei Bedarf auch in Kombination mit anderen antiviralen Wirkstoffen wie beispielsweise Gancyclovir oder Acyclovir eingesetzt werden.

### Biologische Testbeschreibungen:

### in vitro-Wirkung:

Anti-HCMV- (Anti-Humanes Cytomegalie-Virus) und Anti-MCMV- (Anti-Murines Cytomegalie-Virus) Zytopathogenitätstests:
Die Testverbindungen wurden als 50 millimolare (mM) Lösungen in Dimethylsulfoxid (DMSO) eingesetzt. Ganciclovir, Foscarnet und Cidofovir dienten als Referenzverbindungen. Nach der Zugabe von jeweils 2 µl der 50, 5, 0,5 und 0,05 mM DMSO-Stammlösungen zu je 98 µl Zellkulturmedium in der Reihe 2 A-H in Doppelbestimmung wurden 1:2-Verdünnungen mit je 50 µl Medium bis zur Reihe 11 der 96-Well-Platte durchgeführt. Die Wells in den Reihen 1 und 12 enthielten je 50 µl Medium. In die Wells wurden dann je 150 µl einer Suspension von 1 x 10⁴ Zellen (humane Lungenfibroblasten [HELF]) pipettiert (Reihe 1 = Zellkontrolle) bzw. in die Reihen 2-12 ein Gemisch von HCMV-infizierten und nichtinfizierten HELF-Zellen (M.O.I. = 0,001 - 0,002), d.h. 1-2 infizierte Zellen auf 1000 nichtinfizierte Zellen. Die Reihe 12 (ohne Substanz) diente als Viruskontrolle. Die End-Testkonzentrationen lagen bei 250 - 0,0005 µM. Die Platten wurden 6 Tage bei 37°C/5% CO₂ inkubiert, d.h. bis in den Viruskontrollen alle Zellen infiziert waren (100% cytopathogener Effekt [CPE]). Die Wells wurden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach wurden die Platten mit einem Overhead-Mikroskop (Plaque multiplier der Firma Technomara) visuell ausgewertet.

Die folgenden Daten konnten von den Testplatten ermittelt werden:
EC₅₀ (HCMV) = Substanzkonzentration in µM, die den CPE (cytopathischen Effekt) um 50% im Vergleich zur unbehandelten Viruskontrolle hemmt;
SI (Selektivitätsindex) = CC₅₀ (HELF) / EC₅₀ (HCMV).

Der Anti-MCMV-Test wurde in Abweichung zum vorstehend für HCMV beschriebenen Verfahren mit folgenden Veränderungen durchgeführt: Eine zellfreie Virussuspension wurde mit einer konzentrierten Zellsuspension (3T3-Mauszellen) gemischt und 15 Minuten zur Adsorption der Viren inkubiert, bevor auf 1,3 x 10⁵ Zellen/ml mit Medium verdünnt wurde mit einer End-Multiplizität der Infektion (M.O.I.) von 0,05 - 0,1 und mit je 150 µl in die Wells verteilt wurde. Die Inkubationszeit betrug 5 Tage.

Repräsentative Wirkdaten für die erfindungsgemäßen Verbindungen sind in Tabelle 1 wiedergegeben:

**Tabelle 1**

| **Beispiel Nr.** | **HELF CC₅₀ [µM]** | **HCMV EC₅₀ [µM]** | **SI HCMV** | **3T3 CC₅₀ [µM]** | **MCMV EC₅₀ [µM]** | **SI MCMV** |
|---|---|---|---|---|---|---|
| **1** | >250 | 0,028 | >8929 | 18 | 0,035 | 514 |
| **2** | 118 | 0,35 | 337 | 2,5 | 0,05 | 50 |
| **3** | >250 | 0,074 | 3378 | 63 | 0,074 | 851 |

### In vivo-Wirkung:

### MCMV-Letalitätstest:

### Tiere:

2-3 Wochen alte weibliche immunkompetente Mäuse (12-14 g), Stamm Balb/C AnN oder CD1 wurden von kommerziellen Züchtern (Bomholtgaard, Iffa, Credo) bezogen. Die Tiere wurden nicht steril gehalten.

### Virusanzucht:

Murines Cytomegalievirus (MCMV), Stamm Smith, wurde *in vivo* wiederholt in weiblichen CD1-Mäusen passagiert. 21 Tage nach intraperitonealer Infektion (2 x 10⁴ Plaque Forming Units / 0,2ml/Maus) wurden die Speicheldrüsen entnommen, im dreifachen Volumen Minimal Essential Medium (MEM) + 10% foetalem Kälberserum (FKS) aufgenommen und mit Hilfe eines Ultrathurax homogenisiert. Es wurde 10% DMSO v/v zugegeben, 1 ml-Aliquots hergestellt und die Virussuspension bei -140°C aufbewahrt. Nach serieller Verdünnung des Speicheldrüsenisolats in Zehnerschritten erfolgte die Titerbestimmung in Zellkultur auf NIH 3T3-Zellen nach Färbung mit Giemsalösung, sowie die Bestimmung der letalen *Dosis in vivo* in 2-3 Wochen alten Balb/C Mäusen.

### Virusinfektion der Versuchstiere, Behandlung und Auswertung:

2-3 Wochen alte weibliche immunkompetente Balb/C Mäuse (12-14 g) wurden mit 3 x 10⁵ PFU / 0.2ml/Maus intraperitoneal infiziert. 6 Stunden nach der Infektion beginnend wurden die Mäuse über einen Zeitraum von 5 Tagen zweimal täglich (8.00 und 16.00 Uhr) peroral mit Substanz behandelt. Die Dosis betrug 3, 10, 30 oder 90 mg/kg Körpergewicht, das Applikationsvolumen 10 ml/kg Körpergewicht. Die Formulierung der Substanzen erfolgte in Form einer 0,5%-igen Tylosesuspension.mit 2 % DMSO. Im Zeitraum von 4-8 Tagen nach Infektion sterben die placebo-behandelten Kontrolltiere. Die Auswertung erfolgt durch die Ermittlung des Prozentsatzes überlebender Tiere nach Substanzbehandlung im Vergleich zur placebobehandelten Kontrollgruppe.

### HCMV Xenograft-Gelfoam^{®} -Modell:

### Tiere:

3-4 Wochen alte weibliche immundefiziente Mäuse (16-18 g), Fox Chase SCID oder Fox Chase SCID-NOD wurden von kommerziellen Züchtern (Bomholtgaard, Jackson) bezogen. Die Tiere wurden unter sterilen Bedingungen (einschließlich Streu und Futter) in Isolatoren gehalten.

### Virusanzucht:

Humanes Cytomegalievirus (HCMV), Stamm DavisSmith, wurde *in vitro* auf humanen embryonalen Vorhautfibroblasten (NHDF-Zellen) angezüchtet. Nach Infektion der NHDF-Zellen mit einer Multiplizität der Infektion (M.O.I) von 0.01 wurden die virusinfizierten Zellen 5-7 Tage später geerntet und in Gegenwart von Minimal Essential Medium (MEM), 10% foetalem Kälberserum (FKS) mit 10% DMSO bei -140°C aufbewahrt. Nach serieller Verdünnung der virusinfizierten Zellen in Zehnerschritten erfolgte die Titerbestimmung auf 24-Well-Platten konfluenter NHDF-Zellen nach Vitalfärbung mit Neutralrot.

### Vorbereitung der Schwämme, Transplantation, Behandlung und Auswertung:

1x1x1 cm große Kollagenschwämme (Gelfoam^{®}; Fa. Peasel & Lorey, Best.-Nr. 407534; K.T. Chong et al., Abstracts of 39th Interscience Conference on Antimicrobial Agents and Chemotherapy, 1999, S. 439) werden zunächst mit Phosphat-gepufferter Saline (PBS) benetzt, die eingeschlossenen Luftblasen durch Entgasen entfernt und dann in MEM + 10% FKS aufbewahrt. 1 x 10⁶ virusinfizierte NHDF-Zellen (Infektion mit HCMV-Davis M.O.I = 0.01) werden 3 Stunden nach Infektion abgelöst und in 20 µl MEM, 10 % FKS auf einen feuchten Schwamm getropft. 12-13 Stunden später werden die infizierten Schwämme mit 25 µl PBS / 0,1% BSA / 1 mM DTT mit 5 ng/µl basic Fibroblast Growth Factor (bFGF) inkubiert. Zur Transplantation werden die immundefizienten Mäuse mit Avertin narkotisiert, das Rückenfell mit Hilfe eines Trockenrasierers entfernt, die Oberhaut 1-2 cm geöffnet, entlastet und die feuchten Schwämme unter die Rückenhaut transplantiert. Die Operationswunde wird mit Gewebekleber verschlossen. 24 Stunden nach der Transplantation wurden die Mäuse über einen Zeitraum von 8 Tagen zweimal täglich (8.00 und 16.00 Uhr) peroral mit Substanz behandelt. Die Dosis betrug 10 oder 30 mg/kg Körpergewicht, das Applikationsvolumen 10 ml/kg Körpergewicht. Die Formulierung der Substanzen erfolgte in Form einer 0,5%-igen Tylosesuspension mit 2% DMSO. 10 Tage nach Transplantation und 16 Stunden nach der letzten Substanzapplikation wurden die Tiere schmerzlos getötet und der Schwamm entnommen. Die virusinfizierten Zellen wurden durch Kollagenaseverdau (330 U / 1,5 ml) aus dem Schwamm freigesetzt und in Gegenwart von MEM, 10% foetalem Kälberserum, 10% DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Vitalfärbung mit Neutralrot. Ermittelt wurde die Anzahl infektiöser Viruspartikel nach Substanzbehandlung im Vergleich zur placebobehandelten Kontrollgruppe.

Der im folgenden beschriebene Test dient der Untersuchung der erfindungsgemäßen Substanzen im Hinblick auf ihr Nebenwirkungspotential bezüglich einer Induktion von Cytochrom P450-Enzymen.

### Untersuchung der Induktion von Cytochrom P450-Enzymen in humanen Leberzellkulturen:

Primäre humane Hepatozyten wurden mit einer Zelldichte von 2,5 x 10⁵ Zellen zwischen zwei Schichten von Collagen in 24 well-Mikrotiterplatten bei 37°C bei 5 % CO₂ 8 Tage kultiviert. Das Zellkulturmedium wurde täglich gewechselt.

Nach 48 Std. in Kultur wurden die Hepatozyten für 5 Tage in Doppelbestimmung mit unterschiedlichen Konzentrationen der Testsubstanzen im Vergleich mit den Induktoren Rifampicin (50 µM) und Phenobarbital (2 mM) behandelt. Die Endkonzentrationen der Testsubstanzen lagen bei 0,1 - 10 µg/ml.

Von den Zellkulturen wurde der induktive Effekt der Testsubstanzen auf die Cytochrom (CYP) P450-Enzyme 1A2, 2B6, 2C19 und 3A4 durch Zugabe der Substrate 7-Ethoxyresorufin (CYP1A2), [¹⁴C]S-Mephenytoin (CYP2B6 und 2C19) und [¹⁴C]Testosteron (CYP3A4) am Tag 8 bestimmt. Von den so gemessenen Enzymaktivitäten CYP1A2, 2B6, 2C19 und 3A4 behandelter Zellen im Vergleich zu unbehandelten Zellen wurde das induktive Potential der Testsubstanzen ermittelt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral, parenteral oder topisch, insbesondere perlingual oder intravenös.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

### Abkürzungen:

| | |
|---|---|
| Aloc-Cl | Chlorameisensäureallylester |
| DCM | Dichlormethan |
| DIC | N,N'-Diisopropylcarbodiimid |
| DIEA | Diisopropylethylamin |
| DMF | Dimethylformamid |
| eq. | Äquivalente |
| HOAc | Essigsäure |
| HOBt | Hydroxybenzotriazol |
| HONSu | N-Hydroxysuccinimid |
| MTP | Microtiterplatte |
| PS- | Polystyrol-Harz- |
| PyBOP | Benzotriazolyl-N-Oxi-tris(dimethylamino)phosphonium-Hexafluorophosphat |
| Rt | Retentionszeit |
| RT | Raumtemperatur |
| TBABH | Tetrabutylammoniumborhydrid |
| TFA | Trifluoressigsäure |
| THF | Tetrahydrofuran |
| TMOF | Trimethylorthoformiat |

### Allgemeine Arbeitsvorschrift für die Umsetzung von Verbindungen der Formel [A-1] mit Verbindungen der Formel [A-2] (AAV 1):

1,0 eq [A-1] werden in Dioxan gelöst (0,2 M Lösung), mit 2,5 eq. Pyridin versetzt, die Lösung auf 5°C abgekühlt und danach 1,1 eq. [A-2], worin Q vorzugsweise für Chlor steht, als 1,0 M Lösung zugetropft. Der Ansatz wird 30 min. bei 5°C weitergerührt, anschließend die Kühlung entfernt und 16 h bei Raumtemperatur nachgerührt. Der Ansatz wird auf H₂O gegeben, das ausgefallene Produkt abgesaugt, mit H₂O gewaschen und i. Hochvak. getrocknet.

### Allgemeine Arbeitsvorschrift für die Hydrierung von Verbindungen der Formel [A-3] (AAV 2):

0,14 mol der Verbindungen [A-3] werden in 500 ml DMF oder Ethanol gelöst und unter Argon mit einer Suspension von 6,0 g 10 %-igem Pd-C versetzt. Anschließend wird bei 3 bar Wasserstoff-Druck hydriert. Sobald der Umsatz vollständig ist (DC-oder HPLC-Kontrolle), wird der Pd-C-Katalysator abfiltriert und das Lösungsmittel i. Vak. entfernt. Die Rohprodukte der allgemeinen Formel [B-1] werden ohne weitere Reinigung weiter umgesetzt.

### Allgemeine Arbeitsvorschrift für die Sulfonylierung der Verbindungen der allgemeinen Formel [B-1] (AAV 3):

Unter Argon werden 1,0 eq. der Verbindungen [B-1] in Dioxan (0,2 M Lösung) gelöst und mit 2,5 eq. Pyridin versetzt. Nachdem 30 min. bei Raumtemperatur gerührt wurde, werden 1,1 eq. der Verbindungen der allgemeinen Formel [C-1], worin Z vorzugsweise für Chlor steht, gelöst in Dioxan (1,0 M Lösung) zugegeben und das Gemisch 16 h bei Raumtemperatur gerührt. Anschließend wird die Lösung auf H₂O gegeben und dreimal mit DCM extrahiert. Die organische Phase wird mit ges. NaHCO₃-Lösung gewaschen, über Na₂SO₄ getrocknet, filtriert und das Lösungsmittel i. Vak. entfernt. Der Rückstand [C-2] wird i. Hochvak. getrocknet und anschließend ohne weitere Reinigung weiter umgesetzt.

### Allgemeine Arbeitsvorschrift für die Synthese von Verbindungen der allgemeinen Formel [D-1] aus Verbindungen der allgemeinen Formel [C-2] (AAV 4):

Die Verbindungen der Formel [C-2] (1,0 eq.) werden in Ethanol gelöst (0,1 M Lösung), die Lösung mit Hydroxylamin-Hydrochlorid (1,5 eq.) sowie Triethylamin (1,6 eq.) versetzt, anschließend 4 h unter Rückfluß erhitzt sowie 16 h bei Raumtemperatur nachgerührt. Das Lösungsmittel wird i. Vak. entfernt, der Rückstand in Ethylacetat aufgenommen und 3 x mit Wasser extrahiert, die organische Phase über MgSO₄ getrocknet, filtriert und i. Vak. vom Lösungsmittel befreit. Der Rückstand [D-1] wird i. Hochvak. getrocknet.

### Allgemeine Arbeitsvorschrift für die Umsetzung der Verbindungen mit der allgemeinen Formel [D-1] mit Verbindungen [E-1] (AAV 5):

1,0 eq. der Verbindungen der allgemeinen Formel [D-1], 1,05 eq. Carbonsäure [E-1] und 1,1 eq. PyBOP werden in THF vorgelegt (0,1 M Lösung), die Suspension mit 1,1 eq. *N,N-*Diisopropylethylamin versetzt und die resultierende Lösung 16 h bei Raumtemperatur gerührt. Anschließend wird der Ansatz mit 10 ml DCM verdünnt und je einmal mit 1 N HCl, ges. NaHCO₃-Lösung sowie ges. NaCl-Lösung extrahiert. Die organische Phase wird über Na₂SO₄ getrocknet, filtriert und das Lösungsmittel i. Vak. entfernt. Das Rohprodukt wird direkt weiter umgesetzt.

### Allgemeine Arbeitsvorschrift für die Synthese eines 1,2,4-Oxadiazols aus dem gemäß AAV 5 erhaltenen Rohprodukt (AAV 6):

1,0 mmol von gemäß AAV 5 erhaltenem Rohprodukt werden in 10 ml DMF aufgenommen und die Lösung auf 110°C erhitzt. Sobald der Umsatz vollständig ist (DC- oder HPLC-Kontrolle, ca. 2-16 h), wird der Ansatz mit DCM verdünnt und zweimal mit H₂O extrahiert. Die vereinigten wässrigen Phasen werden zweimal mit DCM extrahiert, die organischen Phasen vereinigt und über Na₂SO₄ getrocknet, filtriert und das Lösungsmittel i. Vak. entfernt. Die so erhaltenen Verbindungen der allgemeinen Formel (I) werden durch Chromatographie an Kieselgel (Cyclohexan/Ethylacetat) oder durch präparative HPLC gereinigt.

### Allgemeine Arbeitsvorschrift für die Abspaltung einer Boc-Schutzgruppe (AAV 7):

1.0 mmol des Boc-geschützten Amins werden in 10 ml eines Gemisches TFA/CH₂Cl₂ oder TFA/Dioxan (1:1 v/v) aufgenommen, und die Lösung bei RT gerührt. Sobald der Umsatz vollständig ist (ca. 45 min), wird das Lösungsmittel i. Vak. entfernt, der Rückstand in DCM aufgenommen und zweimal mit ges. NaHCO₃-Lösung extrahiert. Die vereinigten wässerigen Phasen werden zweimal mit CH₂Cl₂ extrahiert, die organischen Phasen vereinigt und über Na₂SO₄ getrocknet, filtriert und das Lösungsmittel i. Vak. entfernt. Das Produkt wird durch Chromatographie an Kieselgel (Cyclohexan/Ethylacetat) oder durch präparative HPLC gereinigt.

### Allgemeine Arbeitsvorschriften für die Synthesen unter Verwendung von polymeren Trägern:

### Allgemeine Arbeitsvorschrift für die Synthese der 1,3,4-Oxadiazole gemäß Schema 4:

Die Reaktionen gemäß Schema 4 wurden an einem polymeren Träger mit dem IRORI-System nach der dem Festphasenchemiker geläufigen "Split & Mix"-Methode mit 4 Carbonsäurechloriden, 24 Carbonsäuren sowie beiden meta- bzw. para-Isomeren des Phenylendiamins bzw. Sulfonsäurechlorids durchgeführt. Dabei wurden die ersten beiden Stufen in einem Kolben durchgeführt, die übrigen Stufen in den IRORI-Mini-Kans (100 mg Harz pro Kan).

### Synthese der Ausgangsharze (I) und (II) für die Synthesen am polymeren Träger entsprechend Schema 4:

### Reduktive Aminierung von Formyl-Harz (Fa. Nova Biochem, 0,78 mmol/g):

In einem Kolben wird das Formyl-Harz (1,0 eq.) in TMOF/DMF (100 ml pro 12,5 g Harz) suspendiert und mit dem Diamin (6,0 eq.) versetzt. Die Suspension wird 16 h bei 40°C geschüttelt und anschließend mit einer frisch angesetzten Lösung von TBABH (4,0 eq.) und HOAc (16,0 eq.) in DMF versetzt. Nach 8 h bei RT wird das Lösungsmittel abfiltriert und das Harz erneut mit der Reduktionslösung versetzt. Nach weiteren 16 h bei RT wird das Lösungsmittel abgesaugt und das Harz (I) jeweils 2 x mit je 200 ml 50%-iger HOAc, DMF, THF sowie DCM gewaschen und i. Hochvak. getrocknet.

### Sulfonylierung von polymergebundenem Phenylendiamin:

Das Harz (I) (1,0 eq.) wird in THF aufgenommen und mit dem Sulfonsäurechlorid (1,5 eq.) versetzt. Die Suspension wird 16 h bei RT geschüttelt und das Lösungsmittel abgesaugt. Anschließend wird das Harz (II) je 2 x mit je 100 ml 50 % iger HOAc, DMF, THF sowie DCM gewaschen und i. Hochvak. getrocknet.

### Harzvorbereitung für das IRORI-System:

Die Harze vom Typ II werden als Suspension (pro 3,0g Harz: 30 ml DMF/DCM 2:1 v/v) in je 96 Mini-Kans verteilt (1 ml Suspension pro Kan), jeweils dreimal mit DCM gewaschen und die Kans i. Vak. getrocknet.

### Reaktionssequenz (IRORI):

### Acylierung mit Säurechloriden:

Die Kans werden sortiert, in THF aufgenommen und mit 5,0 eq. DIEA sowie 5,0 eq. Säurechlorid versetzt, kurz evakuiert, und 3 h bei RT geschüttelt. Danach werden die Reaktionslösungen abgetrennt, die Kans vereinigt und gewaschen (je 2 x 50 %-ige HOAc, DMF, THF, DCM).

### Hydrazid-Synthese:

Die vereinigten Kans werden in einem Gemisch 2 N NaOH / MeOH / THF (5:7:15 v/v) aufgenommen, kurz evakuiert, und 5 h bei 50°C gerührt. Anschließend werden die Kans gewaschen (je 2 x 50%-ige HOAc, DMF, THF, DCM) und i. Vak. getrocknet. Danach werden die Kans mit THF aufgenommen, mit 5 eq. DIC sowie 10 eq. HONSu versetzt und 3 h bei RT geschüttelt. Es wird abfiltriert, 2 x mit THF gewaschen und anschließend erneut mit THF aufgenommen und mit 3 eq. Hydrazinhydrat versetzt. Nach weiteren 3 h bei RT wird abgesaugt und die Kans mit je 2 x 50%-ige HOAc, DMF, THF, DCM, gewaschen.

### Acylierung mit Carbonsäuren / DIC / HOBt:

Die Carbonsäuren (3 eq.) werden in THF mit 3 eq. DIC, 6 eq. DIEA sowie 6 eq. HOBt versetzt. Nach 60 min Aktivierung bei RT wird die Lösung zu den zuvor sortierten Kans gegeben und 16 h bei RT geschüttelt. Anschließend werden die Kans vereinigt, gewaschen (je 2 x 50%-ige HOAc, DMF, THF, DCM) und i. Vak. getrocknet.

### Cyclisierung zum 1,3,4-Oxadiazol:

Die vereinigten Kans werden in DMF aufgenommen, mit DIC (10 eq.) versetzt, kurz evakuiert und 48 h bei 110°C gerührt. Anschließend werden die Kans gewaschen (je 2 x 50%-ige HOAc, DMF, THF, DCM) und i. Vak. getrocknet.

### Abspaltung vom polymeren Träger:

Nach dem Sortieren in IRORI-Abspaltblöcke werden die Kans aufgeschnitten, das Harz in FlexChem-Blöcke verteilt und die Produkte mit je 1,0 ml TFA/DCM (1:1 v/v) 45 min. bei RT in eine Deep-Well-MTP abgespalten. Das Harz wird mit 1 ml DCM nachgewaschen und das Lösungsmittel eingedampft.

### Ausgangsverbindungen:

### Beispiel I

### 1-Methyl-N-(3-nitrophenyl)-cyclopropanamid

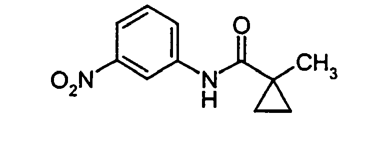

Diese Verbindung wird gemäß AAV 1 aus 80,0 g 3-Nitroanilin hergestellt. Ausbeute: 107 g (81 % d.Th.)

### Beispiel II

### 3-Fluor-2,2-dimethyl-N-(3-aminophenyl)-propanamid

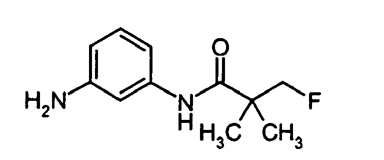

Diese Verbindung wird gemäß AAV 1 und AAV 2 aus 3-Nitroanilin ohne Reinigung der Zwischenstufe hergestellt.
Ausbeute: 85 % d. Th. (über 2 Stufen)

### Beispiel III

### 1-Methyl-N-(3-aminophenyl)-cyclopropanamid

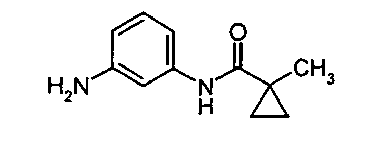

Diese Verbindung wird gemäß AAV 2 aus 107 g der Verbindung aus Beispiel I hergestellt.
Ausbeute: 80 g (87 % d. Th.)

### Beispiel IV

### 3-Fluor-2,2-dimethyl-N-(3-{[(4-methylphenyl)sulfonyl]amino}phenyl)-propanamid

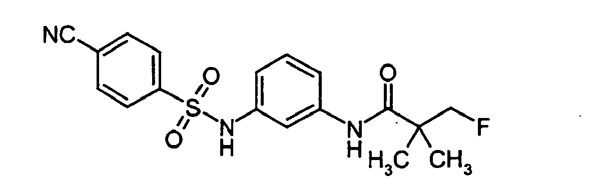

Diese Verbindung wird gemäß AAV 3 aus 18,68 g der Verbindung aus Beispiel II hergestellt.
Ausbeute: 19,96 g (78 % d.Th.)

### Beispiel V

### N-{3-[({4-[Amino(hydroxyimino)methyl]phenyl}sulfonyl)amino]phenyl}-3-fluor-2,2-dimethylpropanamid

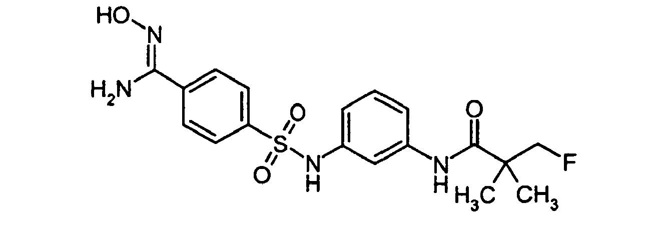

Diese Verbindung wird gemäß AAV 3 aus 10,0 g der Verbindung aus Beispiel IV hergestellt.
Ausbeute: 10,5 g (97 % d. Th.)

### Beispiel VI

### N-(3-{[(4-cyanophenyl)sulfonyl]amino}phenyl)-1-methylcyclopropancarboxamid

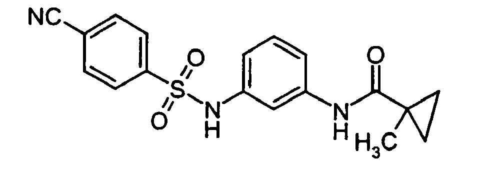

Diese Verbindung wird gemäß AAV 3 aus 80.0 g der Verbindung aus Beispiel III hergestellt.
Ausbeute: 159 g Rohprodukt (>100 % d.Th.)

### Beispiel VII

### N- {3-[({4-[Amino(hydroxyimino)methyl]phenyl}sulfonyl)amino]phenyl}-1-methylcyclopropancarboxamid

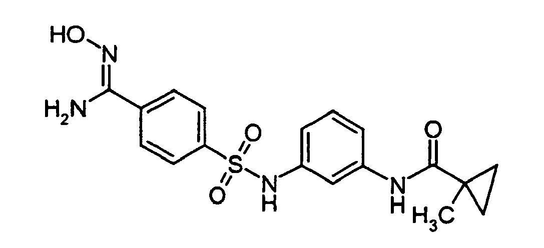

Diese Verbindung wird gemäß AAV 3 aus 158 g der Verbindung aus Beispiel VI hergestellt.
Ausbeute: 148 g (83 % d. Th.)

### Ausführungsbeispiele:

Die im folgenden aufgeführten Ausführungsbeispiele zu 3-verknüpften 1,2,4-Oxadiazolen wurden aus den Verbindungen vom Typ Beispiel V gemäß AAV 5, AAV 6 und AAV 7 hergestellt.

### Beispiel 1

### N-(4-{[(3-{5-[(1S)-1,5-Diaminopentyl]-1,2,4-oxadiazol-3-yl}phenyl)sulfonyl]-amino}phenyl)-1-methylcyclopropancarboxamid

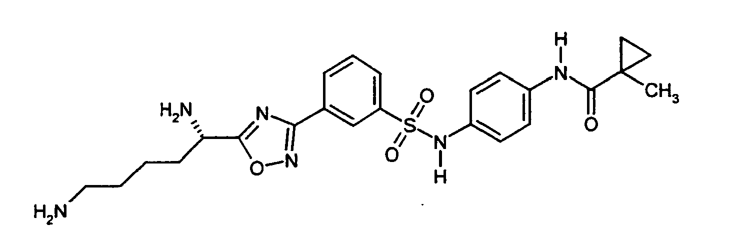

100 mg (0.257 mmol) des Amidoxims, das analog zu Beispiel IV und V hergestellt wird, 93.6 mg (0.27 mmol) Boc-Lys(Boc)-OH und 147 mg (0.27 mmol) PyBOP werden in 3 ml THF vorgelegt, die Suspension bei Raumtemperatur mit 36.6 mg (56.66 mmol) *N,N-*Diisopropylethylamin versetzt und die resultierende klare Lösung 16 h bei Raumtemperatur gerührt. Anschließend wird der Ansatz mit 15 ml CH₂Cl₂ verdünnt und je einmal mit je 10 ml 1N HCl, ges. NaHCO₃-Lösung sowie ges. NaCl-Lösung extrahiert. Die organische Phase wird über Na₂SO₄ getrocknet, filtriert und das Lösungsmittel i. Vak. entfernt. Das Rohprodukt (184 mg) wird in 7 ml DMF aufgenommen und die Lösung für 2.5 h bei 110°C gerührt. Anschließend wird der Ansatz mit 15 ml CH₂Cl₂ verdünnt und die organische Phase zweimal mit je 10 ml H₂O extrahiert. Die vereinigten wässerigen Phasen werden zweimal mit je 10 ml CH₂Cl₂ extrahiert, die organischen Phasen vereinigt und über Na₂SO₄ getrocknet, filtriert und das Lösungsmittel i. Vak. entfernt. Das Produkt wird durch Chromatographie an Kieselgel 60 mit Cyclohexan/Ethylacetat 1:1 v/v gereinigt. Ausbeute : 141 mg (79%), weißer Feststoff.

Zur Abspaltung der Boc-Schutzgruppen wird das Produkt in 5 ml einer TFA/DCM-Lösung (1:1 v/v) gelöst und das Reaktionsgemisch 30 min bei RT gerührt. Anschließend wird das Lösungsmittel i. Vak. entfernt, der Rückstand in 3 ml 1N NaOH aufgenommen, mit 1N HCl auf pH = 9 gestellt und das Rohprodukt auf eine Chromatografie-Säule mit einem schwach sauren Ionentauscher (Amberlite IRC50, 20-50 mesh) gegeben, die Säule mit MeOH/H₂O-Gemischen (1:9 -> 3:6) gewaschen und anschließend das Produkt mit 5% NH₃ in MeOH/H₂O (5:95 v/v) eluiert. Das Lösungsmittel wird i. Vak. entfernt, der Rückstand in 1 ml H₂O aufgenommen und lyophilisiert.
Ausbeute : 15.7 mg (18%), weißes Lyophilisat.
¹H-NMR (200 MHz, DMSO): δ = 0,47-0,60 (m, 2 H), 0,90-1,01 (m, 2 H), 1,22-1,53 (m, 4 H), 1,36 (s, 3 H), 1,65-1,90 (m, 2 H), 2,63 (t, 2 H), 4,12 (t, 1 H), 6,80 (d, 2 H), 7,21 (d, 2 H), 7,59 (t, 1 H), 7,84 (d, 1 H), 8,04 (d, 1 H), 8,33 (s, 1 H), 8,88 (s, 1 H)

### Beispiel 2

### N-(3- {[(4-{5-[(1S)-1-amino-2-methylpropyl]-1,2,4-oxadiazol-3-yl}phenyl)sulfonyl] amino}phenyl)-1-methylcyclopropanecarboxamid

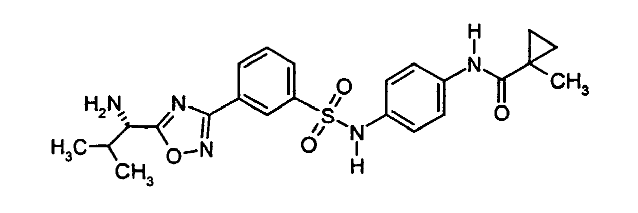

In 15 ml THF werden 183 mg (1.42 mmol) *N,N-*Diisopropylethylamin, 294 mg (1.35 mmol) Boc-Val-OH und 737 mg (1.42 mmol) PyBOP vorgelegt, 30 min bei Raumtemperatur gerührt, dann mit 500 mg (1.29 mmol) des Amidoxims versetzt und die Lösung 16 h bei Raumtemperatur gerührt. Anschließend wird der Ansatz mit 30 ml CH₂Cl₂ verdünnt und je einmal mit je 20 ml 1N HCl, ges. NaHCO₃-Lösung sowie ges. NaCl-Lösung extrahiert. Die organische Phase wird über Na₂SO₄ getrocknet, filtriert und das Lösungsmittel i. Vak. entfernt. Das Rohprodukt (1.18 g) wird in 45 ml DMF aufgenommen und die Lösung für 8 h bei 110°C gerührt. Anschließend wird das Lösungsmittel i. Vak. entfernt, der Rückstand in 20 ml einer TFA/DCM-Lösung (1:1 v/v) gelöst und das Reaktionsgemisch 45 min bei RT gerührt. Anschließend wird das Lösungsmittel i. Vak. entfernt, der Rückstand in 30 ml DCM aufgenommen, die organische Phase zweimal mit je 30 ml H₂O extrahiert. Die vereinigten wässerigen Phasen werden zweimal mit je 30 ml CH₂Cl₂ extrahiert, die organischen Phasen vereinigt und über Na₂SO₄ getrocknet, filtriert und das Lösungsmittel i. Vak. entfernt. Das Produkt wird durch präparative HPLC (CromSil C18, 250x30, Fluß 50 ml/min, Laufzeit: 38 min, Detektion bei 210nm, Gradient 10%ACN (3 min) → 90%ACN (31min) → 90%ACN (34min) -> 10% ACN (34.01 min)) gereinigt.
Ausbeute : 394 mg (65%), weißer Feststoff.
¹H-NMR (300 MHz, DMSO): δ = 0,56-0,62 (m, 2 H), 0,87 (d, 3 H), 0,94 (d, 3 H), 1,01-1,08 (m, 2 H), 1,96-2,12 (m, 1 H), 3,95 (d, 1 H), 6,76 (d, 1 H), 7,11 (t, 1 H), 7,27 (d, 2 H), 7,56 (s, 1 H), 7,94 (d, 2 H), 8,16 (d, 2 H), 9,17 (s, 1 H)

### Beispiel 3

### N-(3-{[(4-{5-[(1S)-1-amino-2-methylpropyl]-1,2,4-oxadiazol-3-yl}phenyl)sulfonyl] amino}phenyl)-3-fluoro-2,2-dimethylpropanamid

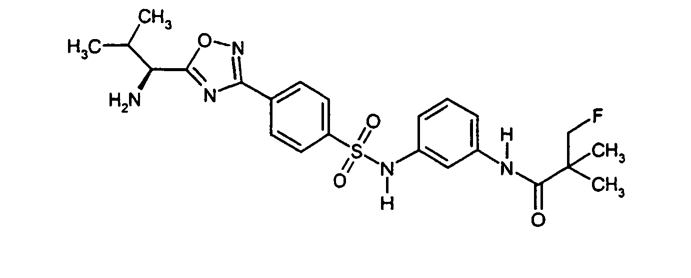

In 50 ml THF werden 522 mg (4.04 mmol) *N,N-*Diisopropylethylamin, 838 mg (3.86 mmol) Boc-Val-OH und 2.1 g (4.04 mmol) PyBOP vorgelegt, 45 min bei Raumtemperatur gerührt, dann mit 1.5 g (3.67 mmol) des Amidoxims versetzt und die Lösung 16 h bei Raumtemperatur gerührt. Anschließend wird der Ansatz i. Vak. eingeengt, der Rückstand mit 200 ml EtOAc aufgenommen und dreimal mit H₂O extrahiert. Die organische Phase wird über Na₂SO₄ getrocknet, filtriert und das Lösungsmittel i. Vak. entfernt. Das Rohprodukt (1.8 g) wird in 45 ml DMF aufgenommen und die Lösung für 4 h bei 110°C gerührt. Anschließend wird der Ansatz mit 100 ml EtOAc verdünnt und dreimal mit H₂O extrahiert. Die organische Phase wird über Na₂SO₄ getrocknet, filtriert und das Lösungsmittel i. Vak. entfernt. Das Produkt wird durch Chromatographie an Kieselgel 60 mit Cyclohexan/Ethylacetat 1:1 v/v gereinigt. Ausbeute : 1498 mg (86%), weißer Feststoff.

Zur Abspaltung der Boc-Schutzgruppen wird das Produkt in 10 ml Dioxan gelöst und mit 10 ml 4N HCl in Dioxan versetzt. Nach 2h bei 60°C wird das Lösunsgmittel i. Vak. entfernt, der Rückstand mit 100 ml ges. NaHCO₃-Lösung versetzt und zweimal mit je 200 ml EtOAc extrahiert. Die organische Phase wird über Na₂SO₄ getrocknet und i. Vak. das Lösungsmittel entfernt. Das Produkt wird i. Hochvak. getrocknet und fällt analysenrein an.
Ausbeute : 910 mg (73%), weißer Feststoff
¹H-NMR (300 MHz, DMSO): δ = 0,86 (d, 3 H), 0,93 (d, 3 H), 1,20 (s, 6 H), 1,96-2,11 (m, 1 H), 3,94 (d, 1 H), 4,47 (d, 2 H), 6,89 (d, 1 H), 7,13 (t, 1 H), 7,30 (d, 1 H), 7,56 (s, 1 H), 7,94 (d, 2 H), 8,26 (d, 2 H), 9,34 (s, 1 H)

| Belspiel | Struktur | MW | HPLC Rt [min] | HPLC Methode/ Instrument | m/z gef. (M+H)* |
|---|---|---|---|---|---|
| 4 | 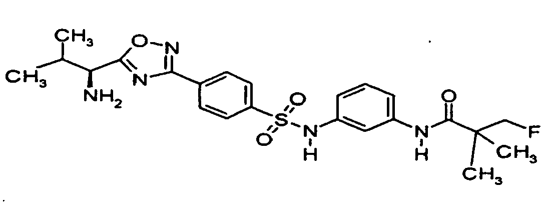 | 489,47 | 4,03 | 1 | 490 |
| 6 | 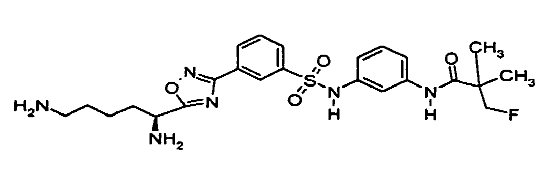 | 518,61 | 2,61 | 3 | 519 |
| 6 | 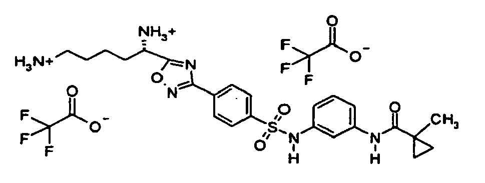 | 726,65 | 3,59 | 1 | 499 |
| 7 | 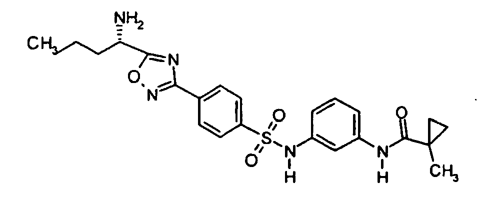 | 483.59 | 2,765 | 2 | 484 |
| 8 | 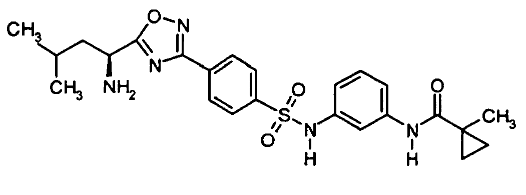 | 483,59 | 1,872 | 2 | 484 |
| 9 | 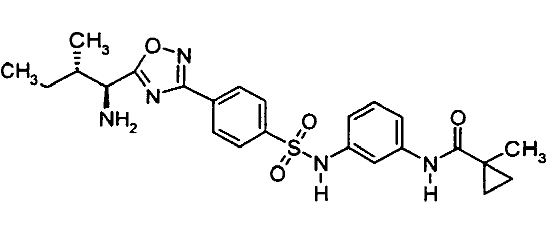 | 483,59 | 1.879 | 2 | 484 |
| 10 | 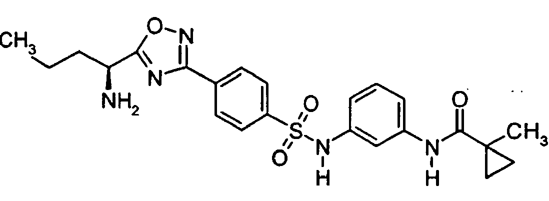 | 469,56 | 1.841 | 2 | 470 |
| 11 | 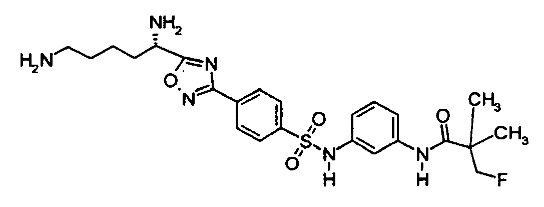 | 518,61 | 3,71 | 1 | 519 |
| 12 | 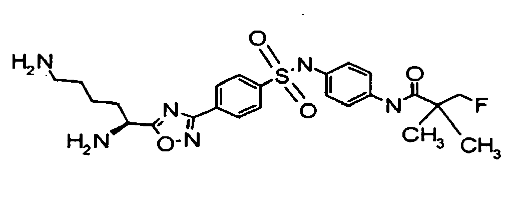 | 518,61 | 3,76 | 1 | 519 |
| 13 | 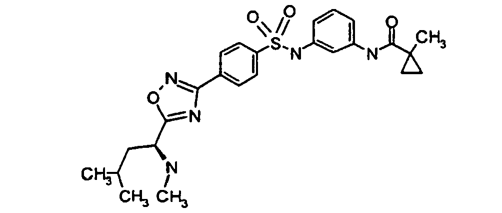 | 497,62 | 3,15 | 3 | 498,1 |
| 14 | 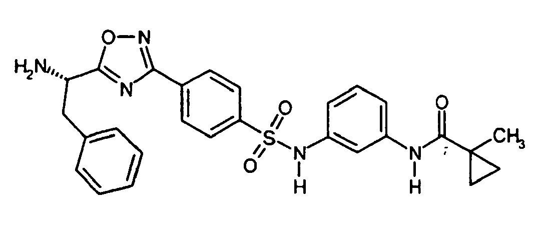 | 517.61 | 2,96 | 4 | 518 |
| 16 | 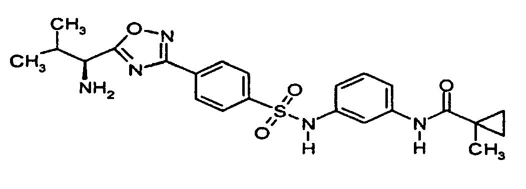 | 518,61 | 3,76 | 1 | 519 |
| 16 | 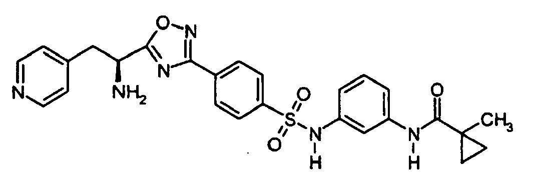 | 518,60 | 2,25 | 4 | 519 |
| 17 | 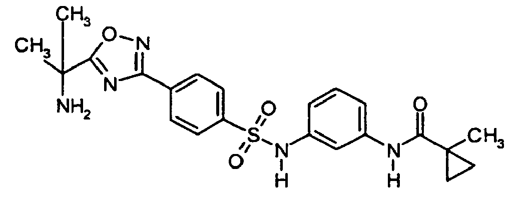 | 455,54 | 1,706 | 2 | 456 |
| 18 | 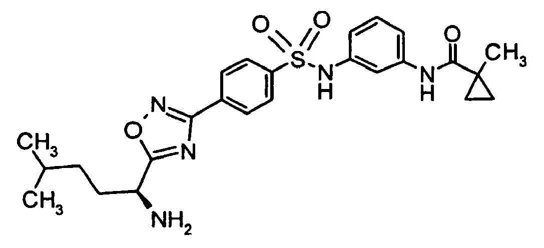 | 497,62 | 3,023 | 3 | 498 |
| 19 | 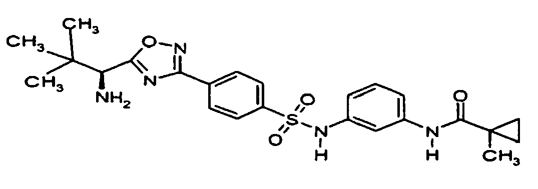 | 483,59 | 2,84 | 4 | 484 |
| 20 | 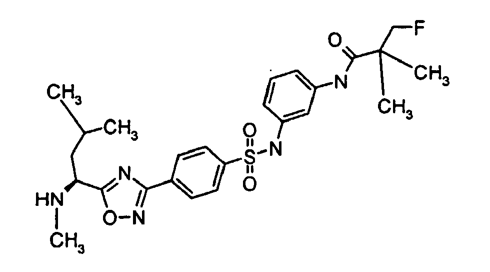 | 518,00 | 4,26 | 1 | 518 |
| 21 | 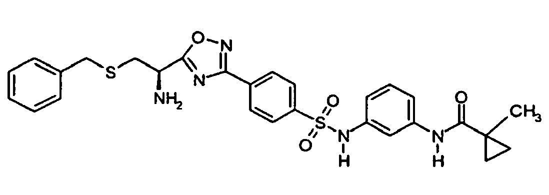 | 563,70 | 3.24 | 3 | 564 |
| 22 | 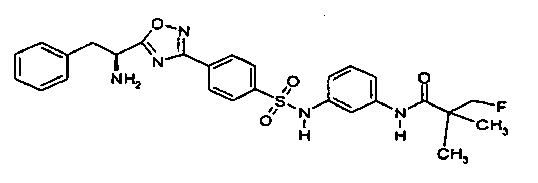 | 537,61 | 4,19 | 1 | 538 |
| 23 | 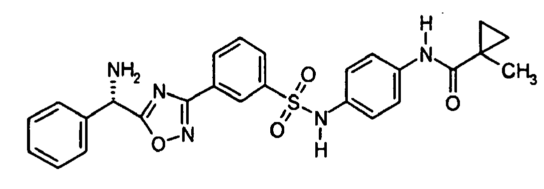 | 503,58 | 4,06 | 1 | 504 |
| 24 | 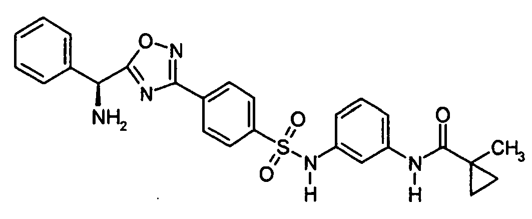 | 503.58 | 1,897 | 2 | 504 |
| 26 | 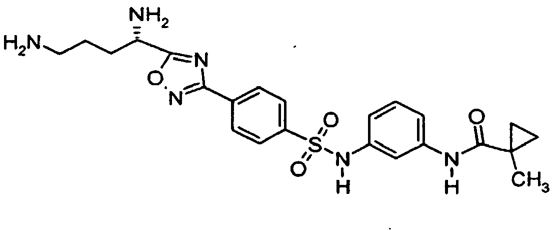 | 484,58 | 2,068 | 2 | 485 |
| 26 | 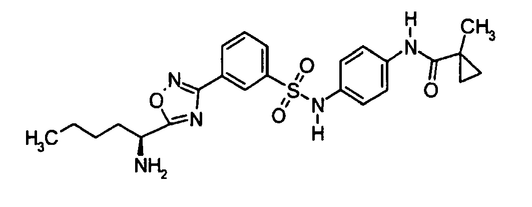 | 483,59 | 2,744 | 2 | 484 |
| 27 | 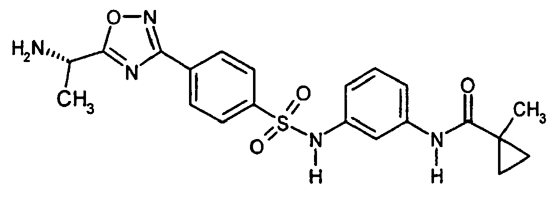 | 441,41 | 2,49 | 3 | 442 |
| 28 | 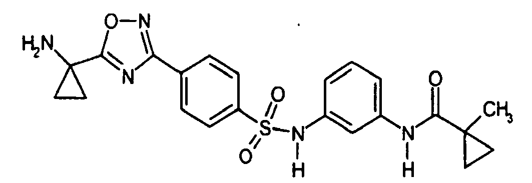 | 453,52 | 2.5 | 3 | 454 |
| 29 | 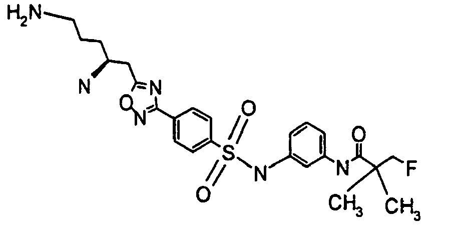 | 518,62 | 3,76 | 1 | 519 |
| 30 | 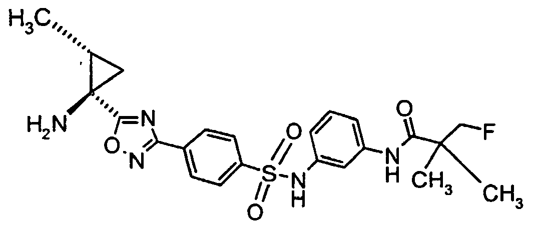 | 487,55 | 4,05 | 1 | 488 |
| 31 | 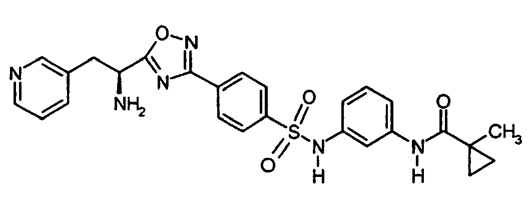 | 518,60 | 1,507 | 2 | 519 |
| 32 | 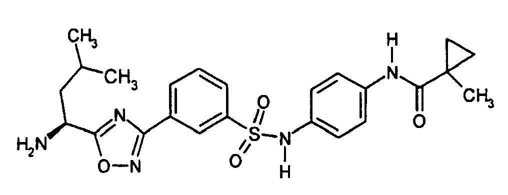 | 483,59 | 4,17 | 1 | 484 |
| 33 | 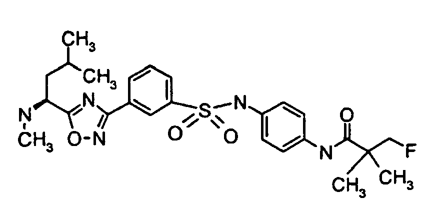 | 518,00 | 4,17 | 1 | 518 |
| 34 | 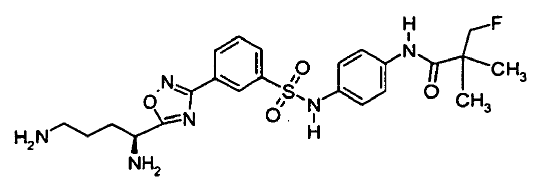 | 504,58 | 3,68 | 1 | 505 |
| 35 | 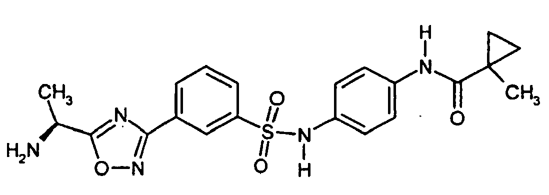 | 441,51 | 3,89 | 1 | 442 |
| 36 | 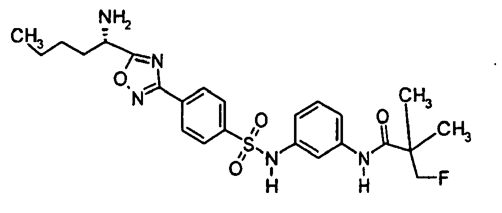 | 503,60 | 4,19 | 1 | 504 |
| 37 | 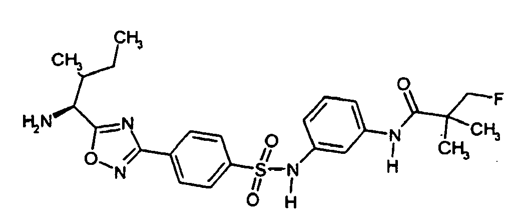 | 503,60 | 4,13 | 1 | 504 |
| 38 | 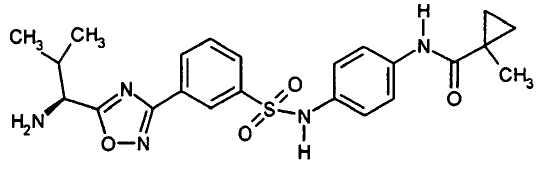 | 483,60 | 4,15 | 1 | 484 |
| 39 | 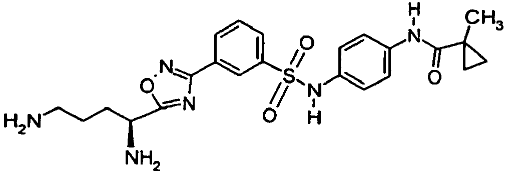 | 484,58 | 1,986 | 2 | 485 |
| 40 | 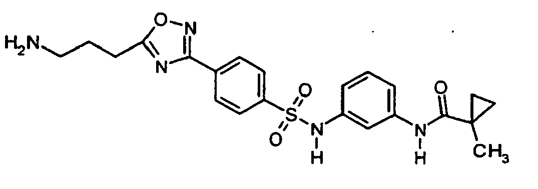 | 455,54 | 2,52 | 2 | 456 |
| 41 | 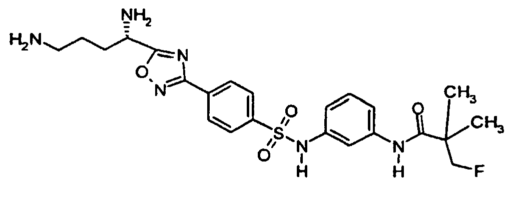 | 504,58 | 3,65 | 1 | 505 |
| 42 | 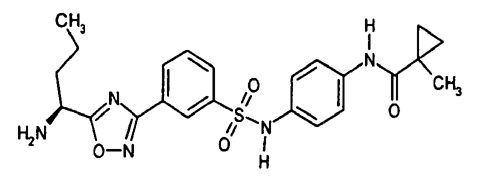 | 469,56 | 4,07 | 1 | 470 |
| 43 | 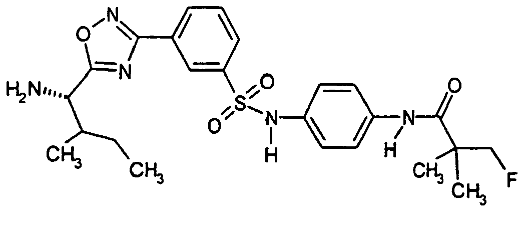 | 503,60 | 4,18 | 1 | 504 |
| 44 | 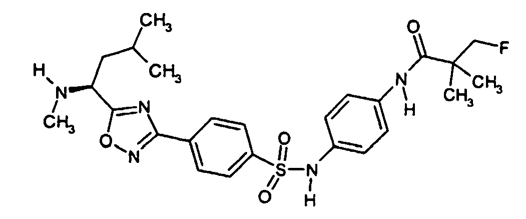 | 517,62 | 4,24 | 1 | 518 |
| 45 | 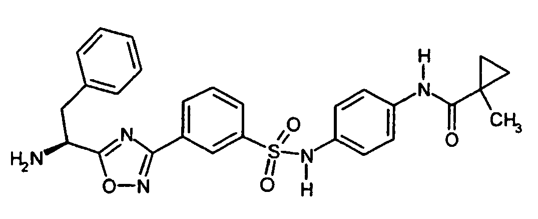 | 517,61 | 4,2 | 1 | 518 |
| 46 | 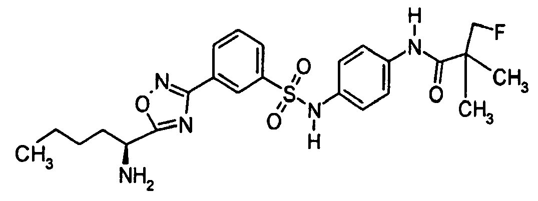 | 503,60 | 4,17 | 1 | 504 |
| 47 | 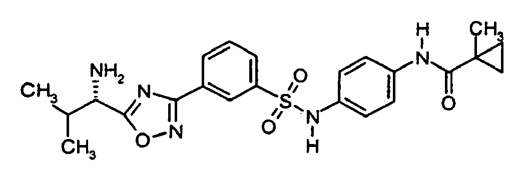 | 469,56 | 3,99 | 1 | 470 |
| 48 | 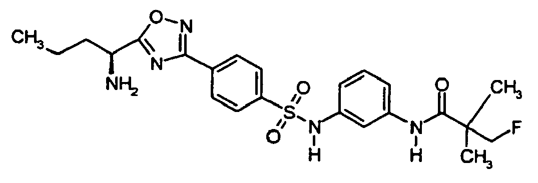 | 489,57 | 4,02 | 1 | 490 |
| 49 | 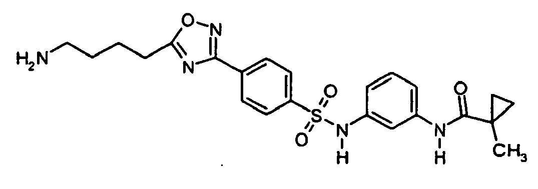 | 469,56 | 3,86 | 1 | 470 |
| 50 | 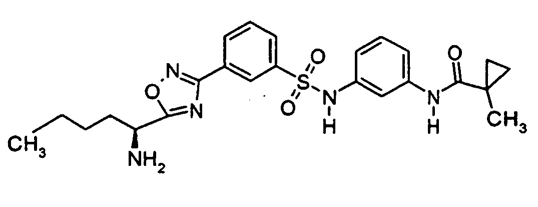 | 483,59 | 2,799 | 2 | 484 |
| 51 | 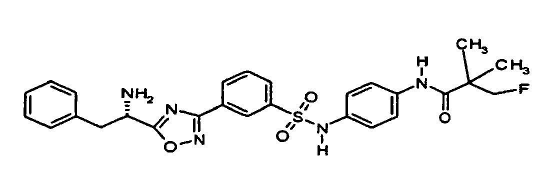 | 537,61 | 4,14 | 1 | 538 |
| 52 | 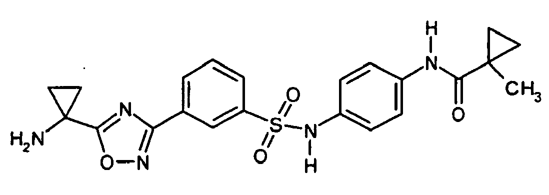 | 453,52 | 3,91 | 1 | 454 |
| 53 | 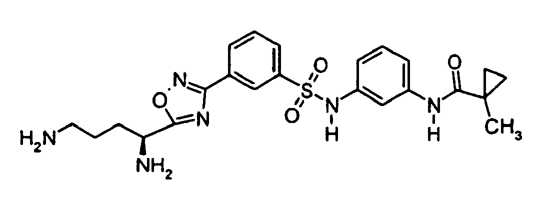 | 484.58 | 2.049 | 2 | 485 |
| 54 | 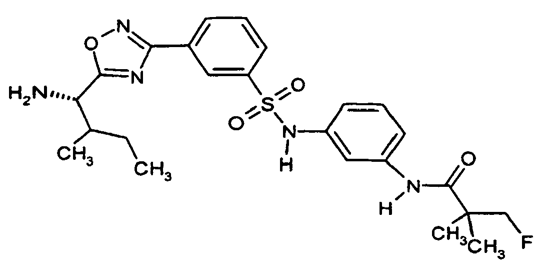 | 503,60 | 4,22 | 1 | 504 |
| 55 | 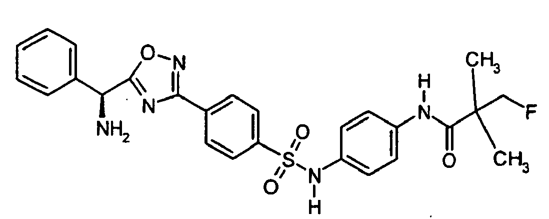 | 523,59 | 4,05 | 1 | 524 |
| 56 | 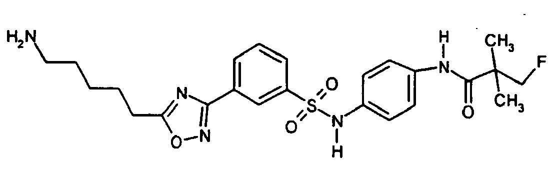 | 503,60 | 3,99 | 1 | 504 |
| 57 | 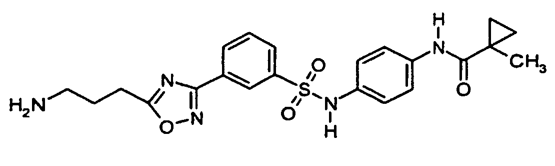 | 455.54 | 2,565 | 2 | 456 |
| 58 | 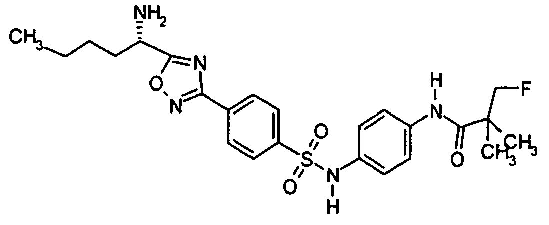 | 503,60 | 2,765 | 2 | 504 |
| 69 | 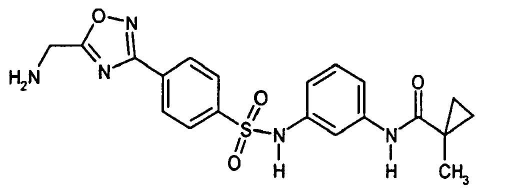 | 427,48 | 2,417 | 2 | 428 |
| 60 | 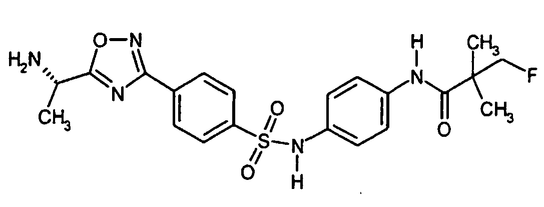 | 461,52 | 3,82 | 1 | 462 |
| 61 | 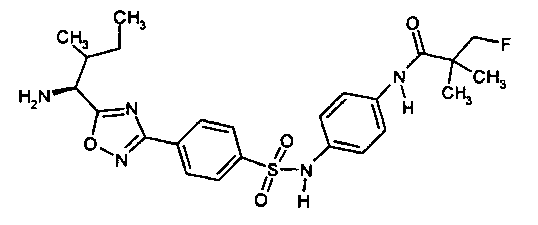 | 503,60 | 4,18 | 1 | 504 |
| 62 | 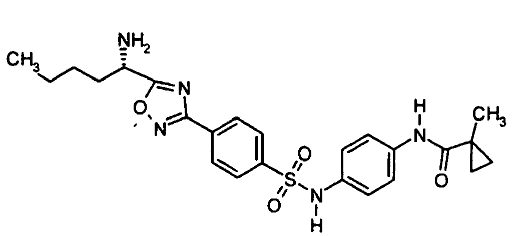 | 483,59 | 2,749 | 2 | 484 |
| 63 | 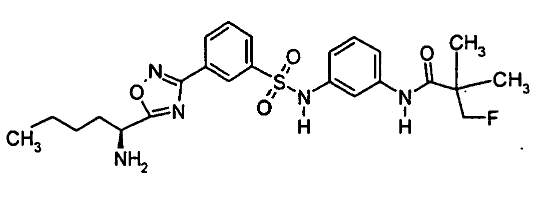 | 503,60 | 2,826 | 2 | 504 |
| 64 | 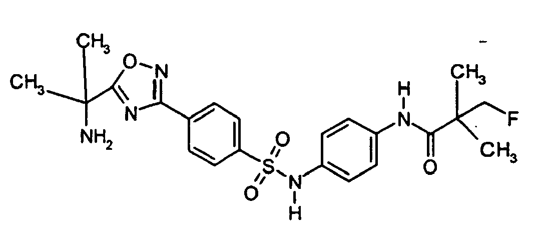 | 475,54 | 3.88 | 1 | 476 |
| 65 | 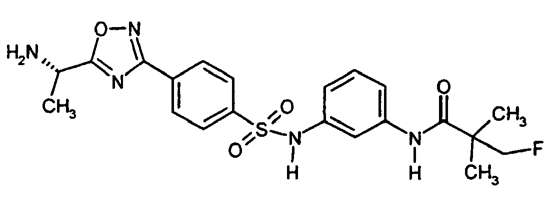 | 461,52 | 3,84 | 1 | 462 |
| 66 | 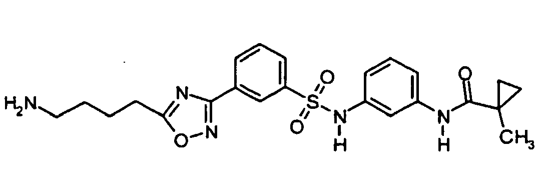 | 469,56 | 2,64 | 2 | 470 |
| 67 | 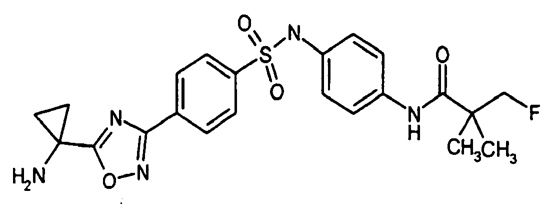 | 473,53 | 3,89 | 1 | 474 |
| 68 | 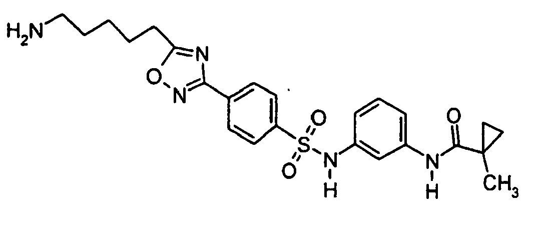 | 483,59 | 2,85 | 4 | 484,5 |
| 69 | 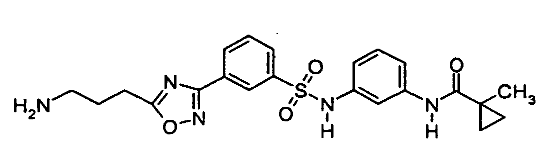 | 455,54 | 2,546 | 2 | 456 |
| 70 | 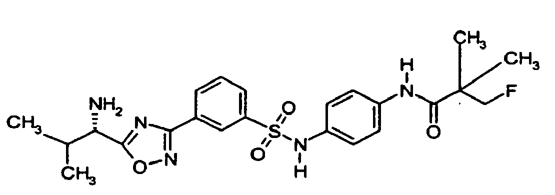 | 489,47 | 3,98 | 1 | 490 |
| 71 | 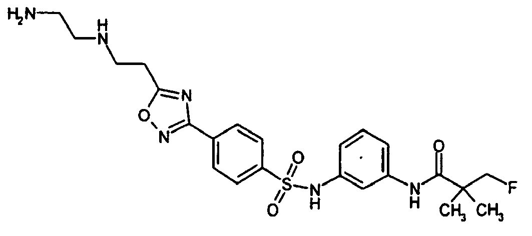 | 504,58 | 3,85 | 1 | 505 |
| 72 | 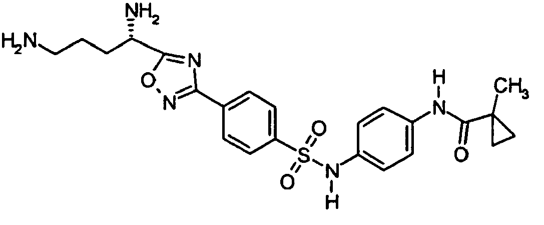 | 484,58 | 1,981 | 2 | 485 |
| 73 | 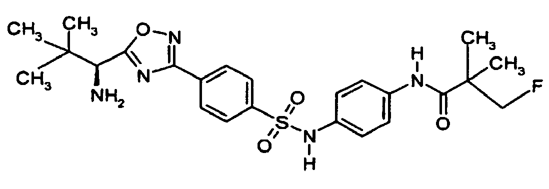 | 503,60 | 4,04 | 1 | 504 |
| 74 | 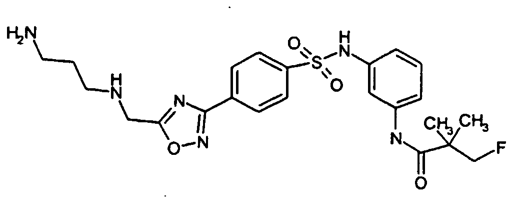 | 504,58 | 3,79 | 1 | 505 |
| 75 | 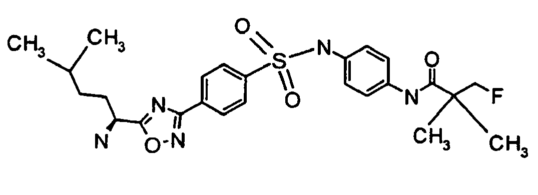 | 517,62 | 4,19 | 1 | 518 |
| 76 | 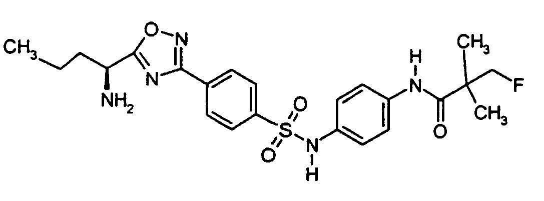 | 489,57 | 4,06 | 1 | 490 |
| 77 | 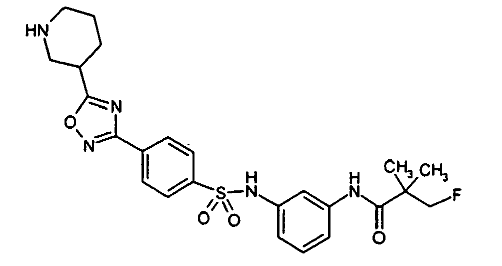 | 501,58 | 3,93 | 1 | 502 |
| 78 | 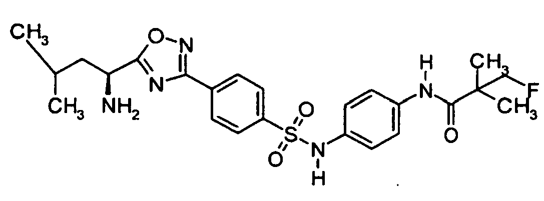 | 503,60 | 4,1 | 1 | 504 |
| 79 | 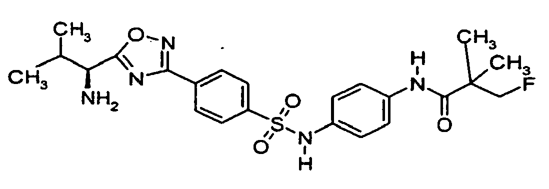 | 489,57 | 3,96 | 1 | 490 |
| 80 | 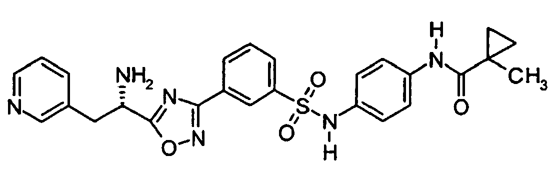 | 518,60 | 3,78 | 1 | 519 |
| 81 | 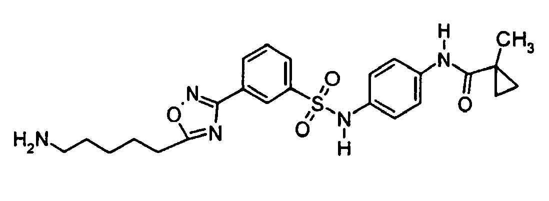 | 483,59 | 2,82 | 4 | 484 |
| 82 | 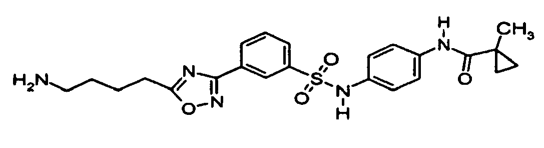 | 469,56 | 2,522 | 2 | 470 |
| 83 | 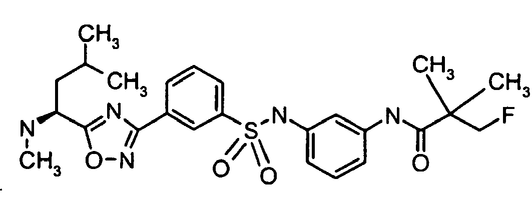 | 518,00 | 4,2 | 1 | 518 |
| 84 | 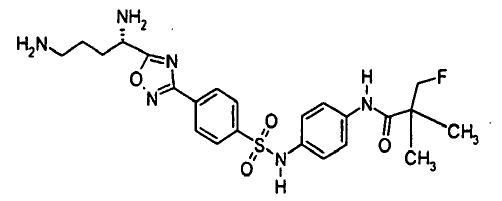 | 504,58 | 2,053 | 2 | 505 |
| 85 | 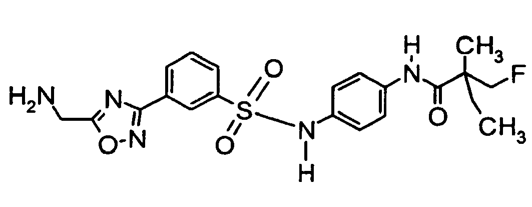 | 447,49 | 3,79 | 1 | 448 |
| 86 | 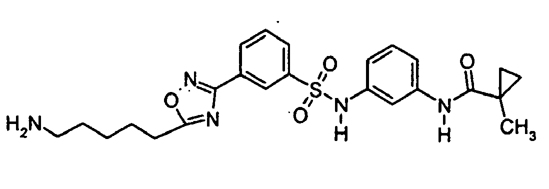 | 483,59 | 2,84 | 4 | 484 |
| 87 | 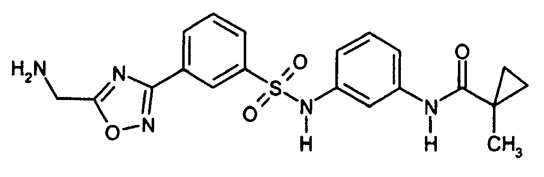 | 427,48 | 2,428 | 2 | 428 |
| 88 | 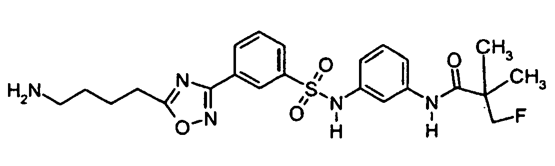 | 489,57 | 2,58 | 2 | 490 |
| 89 | 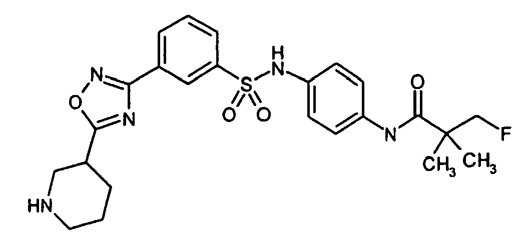 | 501,58 | 3,91 | 1 | 502 |
| 90 | 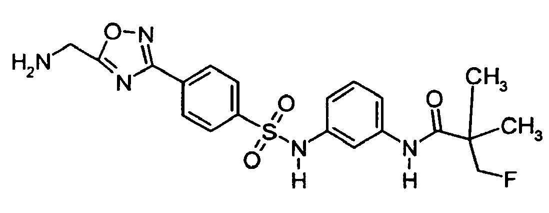 | 447,49 | 3,78 | 1 | 448 |
| 91 | 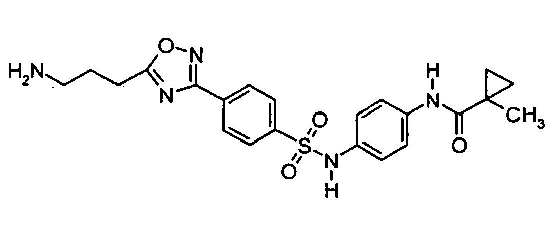 | 455.54 | 2,547 | 2 | 456 |
| 92 | 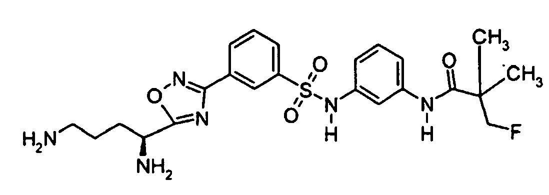 | 504,58 | 2.017 | 2 | 505 |
| 93 | 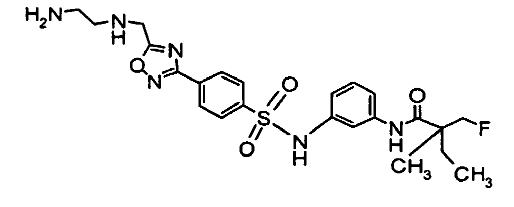 | 490.56 | 3,72 | 1 | 491 |
| 94 | 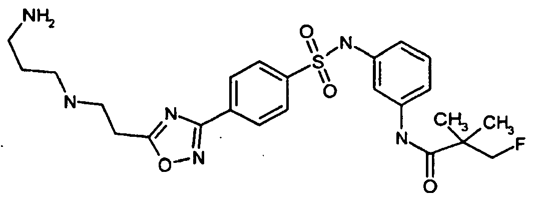 | 518,61 | 3,83 | 1 | 519 |
| 95 | 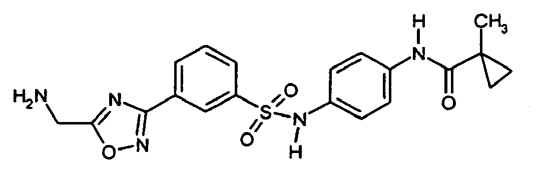 | 427,48 | 2,407 | 2 | 428 |
| 96 | 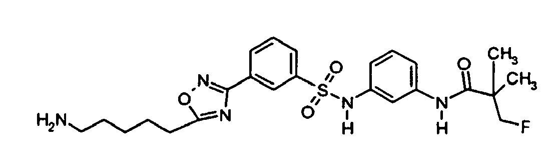 | 503,60 | 2,8 | 4 | 504 |
| 97 | 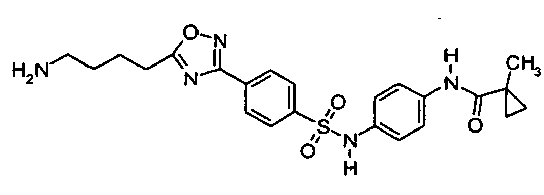 | 469,56 | 2,512 | 2 | 470 |
| 98 | 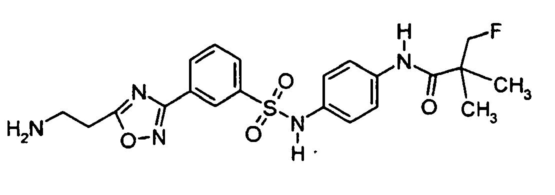 | 461,52 | 2.58 | 4 | 462 |
| 99 | 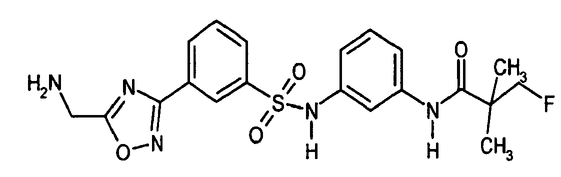 | 447,49 | 2,431 | 2 | 448 |
| 100 | 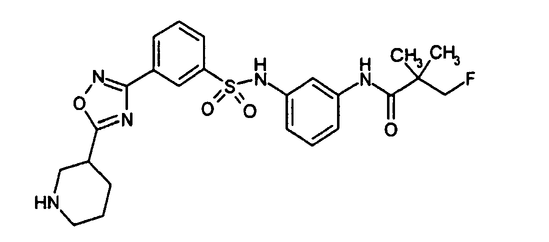 | 501,58 | 3,94 | 1 | 502 |
| 101 | 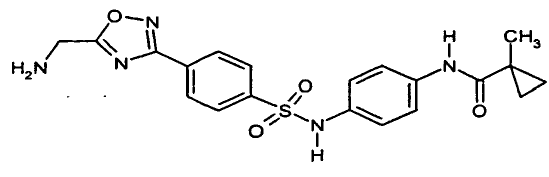 | 427,48 | 2,393 | 2 | 428 |
| 102 | 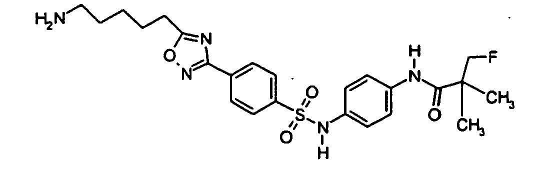 | 503,60 | 2,77 | 4 | 504,5 |
| 103 | 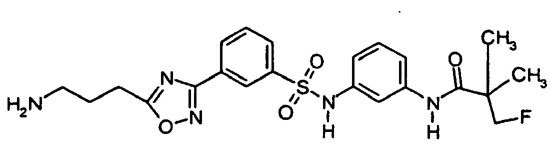 | 475,54 | 2,57 | 2 | 476 |
| 104 | 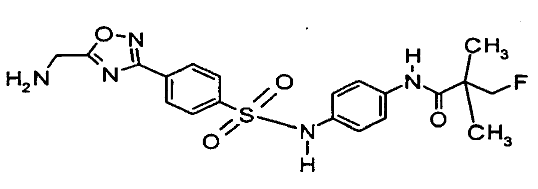 | 447,49 | 2,409 | 2 | 448 |
| 105 | 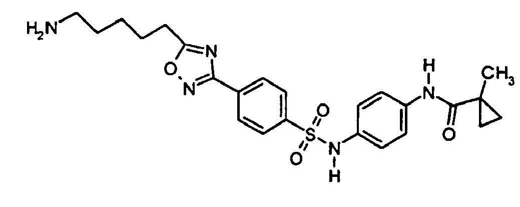 | 483,59 | 2,82 | 4 | 484 |
| 106 | 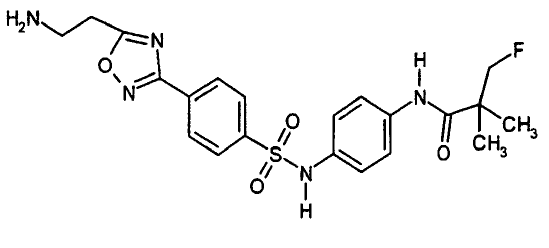 | 461,52 | 2,486 | 2 | 462 |

### Tabelle 2:

Die in den Ausführungsbeispielen und Tabellen aufgeführten Verbindungen wurden unter Anwendung der im folgenden beschriebenen LC-MS- und HPLC-Verfahren charakterisiert:

### Methode 1:

Säule: Kromasil C18 60*2, L-R Temperatur: 30°C, Fluß = 0.75 ml min⁻¹, Eluent: A = 0.005 M HClO₄, B = CH₃CN, Gradient: → 0.5 min 98 % A → 4.5 min 10 % A → 6.5 min 10 % A

### Methode 2:

Säule: Symmetry C18 2.1 x 150 mm, Säulenofen: 50°C, Fluß = 0.9 ml min⁻¹, Eluent: A = 0.3 g 30 %-ige HCl / 1 Wasser, B = CH₃CN, Gradient: 0.0 min 90 % A → 3.0 min 10% A → 6.0 min 10 % A

### Methode 3:

HP1100, Säule: LiChroCart 75-5 LiChrospher 100 RP-18 5 µm, Säulenofen: 40°C, Fluß = 2.5 mlmin⁻¹, Eluent: A = Wasser mit 0.05 % TFA, B = CH₃CN mit 0.05 % TFA, Gradient: 0.0 min 90 % A → 0.05 min 90 % A → 5.0 min 5 % A → 7.0 min 5 % A → 7.05 min 90 % A → 8.0 min 90% A

### Methode 4:

LC-MS: MHZ-2P, Instrument Micromass Platform LCZ Säule: Symmetry C18 50 mm x 2.1 mm, 3.5 µm, Temperatur: 40°C, Fluss = 0.5 ml min⁻¹, Eluent A = CH₃CN + 0.1% Ameisensäure, Eluent B = Wasser + 0.1 % Ameisensäure, Gradient: 0.0 min 10 % A → 4 min 90 % A → 6 min 90 % A

## Patentansprüche

1. Verbindungen der allgemeinen Formel (I) 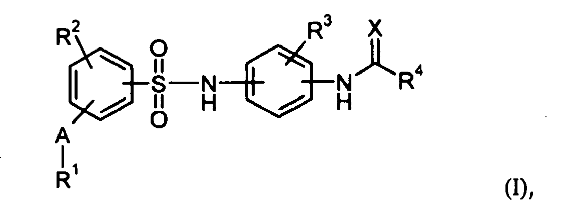 in der
R² und R³ gleich oder verschieden sind und für Wasserstoff, Hydroxy, Halogen, Nitro, Cyano, Trifluormethyl, Trifluormethoxy, (C₁-C₆)-Alkyl, (C₁-C₆)-Alkoxy oder für eine Gruppe der Formel 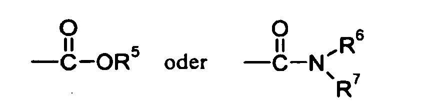 stehen,
in denen
R⁵, R⁶ und R⁷ gleich oder verschieden sind und jeweils für Wasserstoff oder (C₁-C₆)-Alkyl stehen, das seinerseits substituiert sein kann mit ein oder zwei Substituenten, ausgewählt aus der Gruppe bestehend aus Hydroxy, Halogen, Cyano, Trifluormethyl und Trifluormethoxy,
A für über ein C-Atom mit dem benachbarten Phenylring verknüpftes fünf- oder sechsgliedriges Heteroaryl mit bis zu drei Heteroatomen aus der Reihe S, N und/oder O steht,
R¹ für den Rest 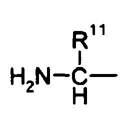 steht, worin
R¹¹ für die Seitengruppe einer Aminosäure steht, und die Ami- nogruppe in R¹ gegebenenfalls ein- oder mehrfach substituiert sein kann mit (C₁-C₆)Alkyl, Alkylcarbonyl, Phenyl,
oder
R¹ für einen geradkettigen oder verzweigten (C₁-C₅)-Alkylrest steht, der seinerseits substituiert sein kann mit einer oder mehreren Gruppen ausgewählt aus Phenyl, Piperidinyl, Pyridinyl, Thiazolyl, Thienyl, 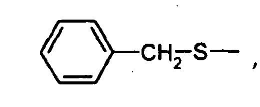 einer Gruppe 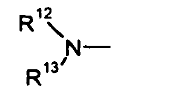
worin
R¹² und R¹³ gleich oder verschieden sind und stehen können für Wasserstoff, (C₁-C₆)Alkyl, Alkylcarbonyl, eine Aminoschutzgruppe, Phenyl,
oder
R¹ für einen Rest 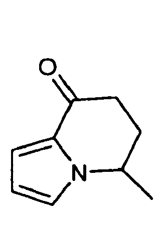 steht, oder
R¹ für einen geradkettigen oder verzweigten (C₁-C₅) Alkylrest steht, der seiner- seits substituiert ist mit einer Gruppe 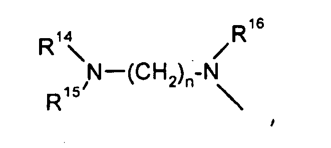
worin
R¹⁴, R¹⁵, R¹⁶ gleich oder verschieden sind und für Wasserstoff oder (C₁-C₆)Alkyl stehen
und
n die Werte 2 oder 3 annehmen kann,
oder
R¹ für Piperidinyl oder den Rest 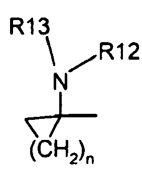 steht, worin R¹² und R¹³ die oben angegebene Bedeutung haben,
n für eine Zahl von 1 bis 4 steht und der Ring bis zu dreifach gleich oder verschieden mit Halogen, (C₁-C₆).Alkyl, Halogen-(C₁-C₆)-Alkyl, Amino, Hydroxy substituiert sein kann,
R⁴ für tert.-Butyl, das gegebenenfalls bis zu dreifach, gleich oder verschie- den, durch Hydroxy, Fluor oder Chlor substituiert ist, oder für Cyclopropyl oder Cyclobutyl steht, die ein- bis dreifach gleich oder unabhängig durch Halogen oder (C₁-C₆)-Alkyl substituiert sind, wobei (C₁-C₆)-Alkyl gegebenenfalls durch Hydroxy, Fluor oder Chlor substitu- iert ist,
und in der
X für Sauerstoff oder Schwefel steht,
und worin stickstoffhaltige Heterocyclen auch als N-Oxide vorliegen können, sowie deren Tautomere, Stereoisomere, stereoisomere Gemische und deren pharmakologisch verträgliche Salze.

2. Verbindungen der allgemeinen Formel (I) gemäß Anspruch 1,
in der
R² und R³ gleich oder verschieden sind und für Wasserstoff oder Halogen stehen,
A für den Rest (A-I) 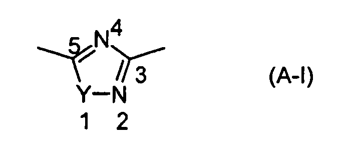
steht, der über eines der Kohlenstoffatome der Positionen 3 oder 5 mit dem benachbarten Phenylring verbunden ist,
und in dem
Y für Sauerstoff steht,
oder
A für den Rest (A-II) 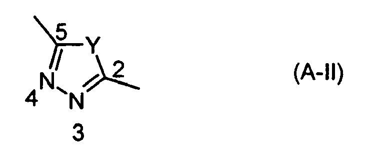
steht, der über eines der Kohlenstoffatome der Positionen 2 oder 5 mit dem benachbarten Phenylring verbunden ist,
und in dem
Y für Sauerstoff steht,
R¹ für den Rest 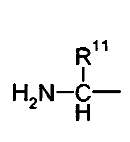 steht,
worin
R¹¹ für die Seitengruppe einer Aminosäure steht, und die Aminogruppe in R¹ gegebenenfalls ein- oder mehrfach substituiert sein kann mit (C₁-C₆)-Alkyl, Alkylcarbonyl, einer Aminoschutzgruppe, Phenyl,
oder
R¹ für einen geradkettigen oder verzweigten (C₁-C₅)-Alkylrest steht, der seinerseits substituiert sein kann mit einer oder mehreren Gruppen
ausgewählt aus Phenyl, Piperidinyl, Pyridinyl, Thiazolyl, Thienyl, 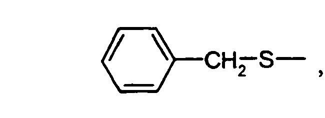 einer Gruppe 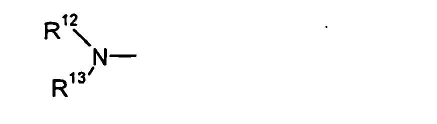 worin
R¹² und R¹³ gleich oder verschieden sind und stehen können für Wasserstoff, (C₁-C₆)-Alkyl, Alkylcarbonyl, eine Aminoschutzgruppe, Phenyl,
oder
R¹ für einen geradkettigen oder verzweigten (C₁-C₅)-Alkylrest steht, der seinerseits substituiert ist mit einer Gruppe 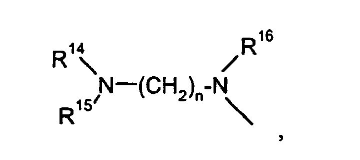
worin
R¹⁴, R¹⁵, R¹⁶ gleich oder verschieden sind und für Wasserstoff oder Methyl stehen
und
n die Werte 2 oder 3 annehmen kann,
oder
R¹ für Piperidin-3-yl oder den Rest steht,
R⁴ für tert.-Butyl, das gegebenenfalls bis zu dreifach, gleich oder verschieden, durch Hydroxy, Fluor oder Chlor substituiert ist, oder für Cyclopropyl oder Cyclobutyl steht, das α-ständig zur Carbonylgruppe oder Thiocarbonylgruppe durch Methyl substituiert ist, welches seinerseits gegebenenfalls durch Hydroxy, Fluor oder Chlor substituiert ist,
und in der
X für Sauerstoff steht,
und worin stickstoffhaltige Heterocyclen auch als N-Oxide vorliegen können, sowie deren Tautomere, Stereoisomere, stereoisomere Gemische und deren pharmakologisch verträglichen Salze.

3. Verbindungen der allgemeinen Formel (I) gemäß Anspruch 1,
in der
R² und R³ für Wasserstoff stehen,
A für einen der Reste 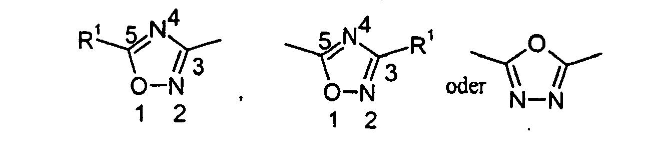
steht,
R¹ für den Rest 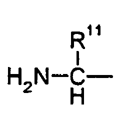 steht, worin
R¹¹ für die Seitengruppe einer Aminosäure steht, und die Aminogruppe in R¹ gegebenenfalls , ein- oder mehrfach substituiert sein kann mit Methyl, Alkylcarbonyl, einer Aminoschutzgruppe, Phenyl,
oder
R¹ für einen geradkettigen oder verzweigten (C₁-C₅)-Alkylrest steht, der seinerseits substituiert sein kann mit einer oder mehreren Gruppen ausgewählt aus Phenyl, Piperidinyl, Pyridinyl, Thiazolyl, Thienyl, 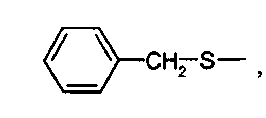 einer Gruppe 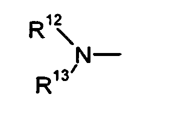 worin
R¹² und R¹³ gleich oder verschieden sind und stehen können für Wasserstoff, Methyl, Alkylcarbonyl, eine Amino- schutzgruppe, Phenyl,
oder
R¹ für einen geradkettigen oder verzweigten (C₁-C₅) Alkylrest steht, der seinerseits substituiert ist mit einer Gruppe
worin
R¹⁴, R¹⁵, R¹⁶ gleich oder verschieden sind und für Wasserstoff oder Methyl stehen
und
n die Werte 2 oder 3 annehmen kann,
oder
R¹ für Piperidin-3-yl oder den Rest
steht,
R⁴ für tert.-Butyl, das gegebenenfalls bis zu dreifach, gleich oder verschieden, durch Hydroxy, Fluor oder Chlor substituiert ist, oder für Cyclopropyl oder Cyclobutyl steht, das α-ständig zur Carbonylgruppe oder Thiocarbonylgruppe durch Methyl substituiert ist, welches seinerseits gegebenenfalls durch Hydroxy, Fluor oder Chlor substituiert ist,
und in der
X für Sauerstoff steht,
und worin stickstoffhaltige Heterocyclen auch als N-Oxide vorliegen können, sowie deren Tautomere, Stereoisomere, stereoisomere Gemische und deren pharmakologisch verträglichen Salze.

4. Verbindungen der allgemeinen Formel (I) gemäß Anspruch 1,
in denen
R⁴ für einen der Reste 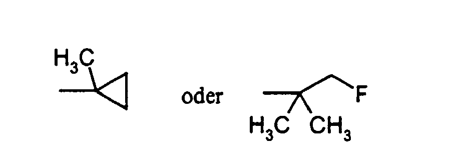
steht.

5. Verbindungen der allgemeinen Formel (Ia) 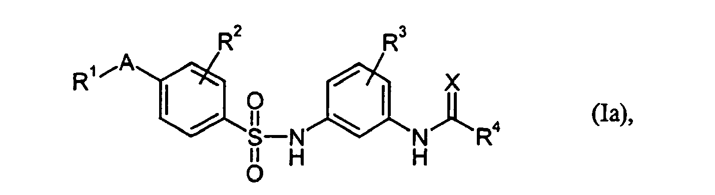 in der
R¹, R², R³, R⁴, A und X die oben angegebenen Bedeutungen haben.

6. Verbindungen der allgemeinen Formel (I) gemäß Anspruch 1, in denen
A für ein 3-verknüpftes 1,2,4-Oxadiazol steht.

7. Verbindungen der allgemeinen Formel (I) gemäß Anspruch 1, die aus der Gruppe der folgenden Verbindungen ausgewählt werden:

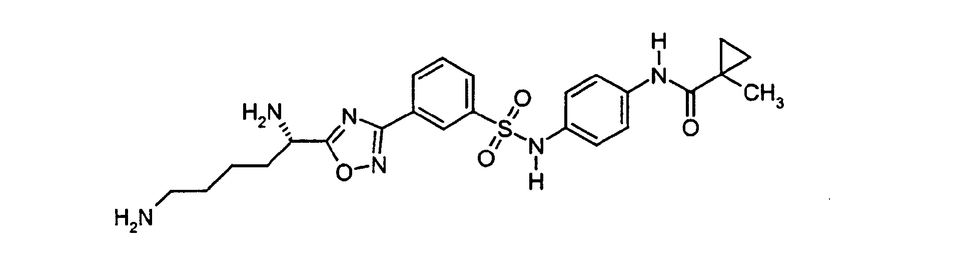

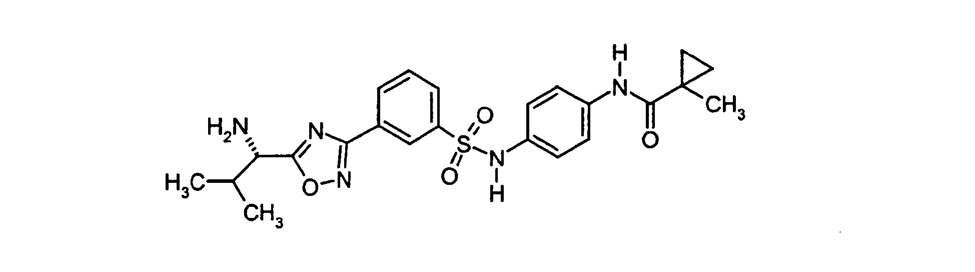

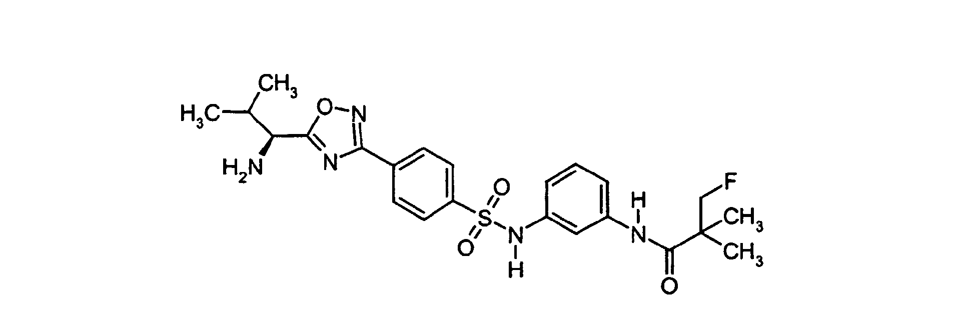

8. Verfahren zur Herstellung von Verbindungen der allgemeinen Formel (I) gemäß Anspruch 1, worin Verbindungen der allgemeinen Formel [D-1] 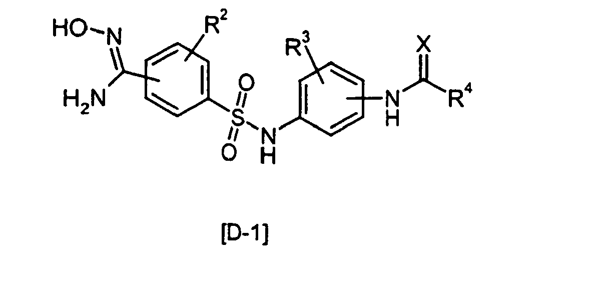 worin
X, R², R³ und R⁴ eine der oben angegebenen Bedeutungen haben, mit einer Carbonsäure der allgemeinen Formel [E-1]
R¹-COOH [E-1]
worin
R¹ die oben angegebene Bedeutung hat und in R¹ enthaltene freie Amino- Gruppen mit Aminoschutzgruppen geschützt vorliegen,
in Gegenwart eines Kondensationsmittels und einer Base acyliert, und das acylierte Amidoxim zum 1,2,4-Oxadiazol cyclisiert.

9. Verfahren zur Herstellung von Verbindungen der allgemeinen Formel (I) gemäß Anspruch 1,
worin Verbindungen der allgemeinen Formel [G-2] 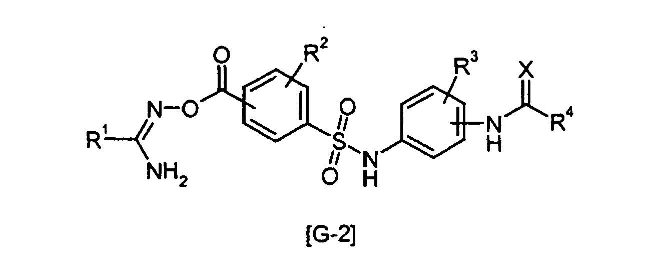 worin
R¹, R², R³, R⁴ und X die oben angegebene Bedeutung haben, cyclisiert werden.

10. Verbindungen der allgemeinen Formel [D-1] 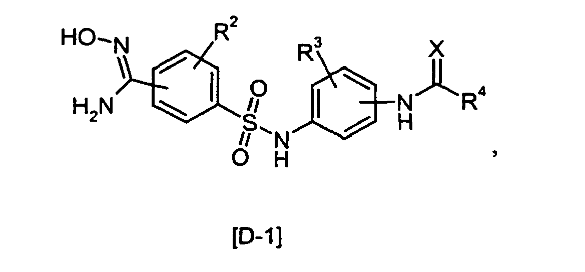 worin R², R³, R⁴ und X die in Anspruch 1 angegebene Bedeutung haben.

11. Verbindungen der allgemeinen Formel [G-2] 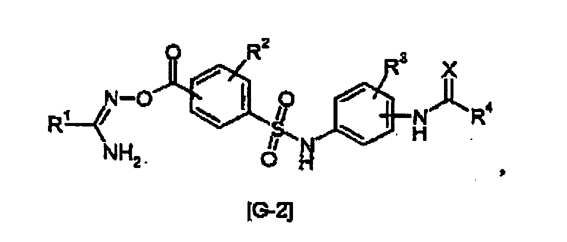 worin R¹, R², R³, R⁴ und X die in Anspruch 1 angegebene Bedeutung haben.

12. Verwendung von Verbindungen der allgemeinen Formel (I) gemäß irgendeinem der Ansprüche 1, bis 7 zur Herstellung von Arzneimitteln.

13. Verwendung von Verbindungen der allgemeinen Formel (I) nach Anspruch 12, worin die Arzneimittel zur Bekämpfung viraler Erkrankungen sind.

14. Verwendung von Verbindungen der allgemeinen Formel (I) nach Anspruch 13, worin die Arzneimittel zur Bekämpfung von Cytomegalievirus-Infektionen sind.

15. Arzneimittel eathaltend Verbindungen der allgemeinen Formel (I) gemäß irgendeimem der Ansprüche 1 bis 7.

## Claims

1. Compounds of general formula (I) 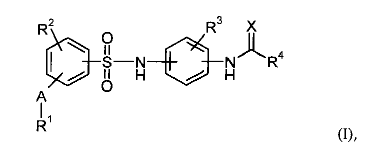 in which
R² and R³ are identical or different and represent hydrogen, hydroxy, halogen, nitro, cyano, trifluoromethyl, trifluoromethoxy, (C₁-C₆)-alkyl, (C₁-C₆)-alkoxy or a group of formula 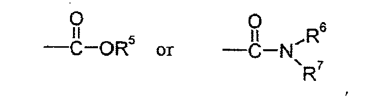 in which
R⁵, R⁶ and R⁷ are identical or different and in each case represent hydrogen or (C₁-C₆)-alkyl, which for its part can be substituted with one or two substituents, selected from the group consisting of hydroxy, halogen, cyano, trifluoromethyl and trifluoromethoxy,
A represents a five- or six-membered heteroaryl having up to three het- eroatoms from the series S, N and/or O linked via a C atom to the adja- cent phenyl ring,
R¹ represents the radical 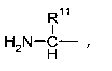
in which
R¹¹ represents the side group of an amino acid, and the amino group in R¹ may optionally be mono- or polysubstituted with (C₁- C₆)alkyl, alkylcarbonyl, phenyl,
or
R¹ represents a straight-chain or branched (C₁-C₅)-alkyl radical, which for its part can be substituted with one or more groups selected from phenyl, piperidinyl, pyridinyl, thiazolyl, thienyl, 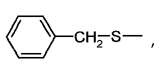 a group 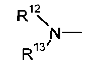 in which
R¹² and R¹³ are identical or different and can represent hydrogen, (C₁-C₆)alkyl, alkylcarbonyl, an amino protecting group, phenyl,
or
R¹ represents a radical 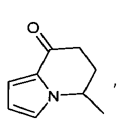 or
R¹ represents a straight-chain or branched (C₁-C₅)-alkyl radical, which for its part is substituted with a group
in which
R¹⁴, R¹⁵, R¹⁶ are identical or different and represent hydrogen or (C₁-C₆)alkyl
and
n can assume the values 2 or 3,
or
R¹ represents piperidinyl or the radical in which R¹² and R¹³ have the meaning indicated above,
n represents a number from 1 to 4 and the ring can be substituted up to three times in an identical or different manner with halogen, (C₁-C₆)- alkyl, halogeno-(C₁-C₆)-alkyl, amino, hydroxy,
R⁴ represents tert-butyl, which is optionally substituted up to three times, in an identical or different manner, with hydroxy, fluorine or chlorine, or
represents cyclopropyl or cyclobutyl, which are substituted up to three times with an identical or independent manner with halogen or (C₁-C₆)-alkyl, with (C₁-C₆)-alkyl optionally being substituted with hydroxy, fluorine or chlorine,
and in which
X represents oxygen or sulphur,
and in which nitrogen-containing heterocycles can also be present as N-oxides,
and their tautomers, stereoisomers, stereoisomeric mixtures and their pharmacologically acceptable salts.

2. Compounds of general formula (I) according to Claim 1,
in which
R² and R³ are identical or different and represent hydrogen or halogen,
A represents the radical (A-I) 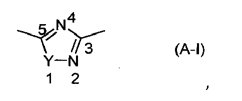
which is linked via one of the carbon atoms of positions 3 or 5 to the adjacent phenyl ring,
and in which
Y represents oxygen,
or
A represents the radical (A-II) 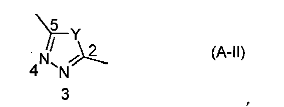
which is linked via one of the carbon atoms of positions 2 or 5 to the adjacent phenyl ring,
and in which
Y represents oxygen,
R¹ represents the radical 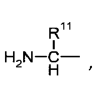 in which
R¹¹ represents the side group of an amino acid, and the amino group in R¹ can optionally be mono- or polysubstituted with (C₁-C₆)- alkyl, alkylcarbonyl, an amino protecting group, phenyl,
or
R¹ represents a straight-chain or branched (C₁-C₅)-alkyl radical, which for its part can be substituted with one or more groups selected from phenyl, piperidinyl, pyridinyl, thiazolyl, thienyl, 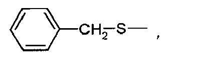
a group 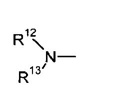 in which
R¹² and R¹³ are identical or different and can represent hydrogen, (C₁- C₆)-alkyl, alkylcarbonyl, an amino protecting group, phenyl,
or
R¹ represents a straight-chain or branched (C₁-C₅)-alkyl radical, which for its part is substituted with a group 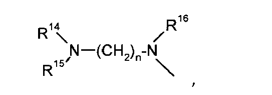
in which
R¹⁴ R¹⁵, R¹⁶ are identical or different and represent hydrogen or methyl
and
n can assume the values 2 or 3,
or
R¹ represents piperidin-3-yl or the radical 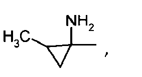
R⁴ represents tert-butyl, which is optionally substituted up to three times, in an identical or different manner, with hydroxy, fluorine or chlorine, or represents cyclopropyl or cyclobutyl, which is substituted in the α- position to the carbonyl group or thiocarbonyl group with methyl, which for its part is optionally substituted with hydroxy, fluorine or chlorine,
and in which
X represents oxygen,
and in which nitrogen-containing heterocycles can also be present as N-oxides,
and their tautomers, stereoisomers, stereoisomeric mixtures and their pharmacologically acceptable salts.

3. Compounds of general formula (I) according to Claim 1,
in which
R² and R³ represent hydrogen,
A represents one of the radicals 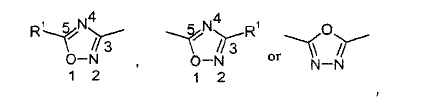
R¹ represents the radical 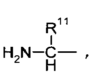
in which
R¹¹ represents the side group of an amino acid, and the amino group in R¹ can optionally be mono- or polysubstituted with methyl, alkylcarbonyl, an amino protecting group, phenyl,
or
R¹ represents a straight-chain or branched (C₁-C₅)-alkyl radical, which for its part can be substituted with one or more groups selected from phenyl, piperidinyl, pyridinyl, thiazolyl, thienyl, 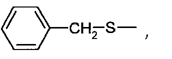 a group 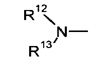 in which
R¹² and R¹³ are identical or different and can represent hydrogen, methyl, alkylcarbonyl, an amino protecting group, phenyl,
or
R¹ represents a straight-chain or branched (C₁-C₅) alkyl radical, which for its part is substituted with a group
in which
R¹⁴, R¹⁵, R¹⁶ are identical or different and represent hydrogen or methyl
and
n can assume the values 2 or 3,
or
R¹ represents piperidin-3-yl or the radical
R⁴ represents tert-butyl, which is optionally substituted up to three times, in an identical or different manner, with hydroxy, fluorine or chlorine, or represents cyclopropyl or cyclobutyl, which is substituted in the α- position to the carbonyl group or thiocarbonyl group with methyl, which for its part is optionally substituted with hydroxy, fluorine or chlorine,
and in which
X represents oxygen,
and in which nitrogen-containing heterocycles can also be present as N-oxides,
and their tautomers, stereoisomers, stereoisomeric mixtures and their pharmacologically acceptable salts.

4. Compounds of general formula (I) according to Claim 1,
in which
R⁴ represents one of the radicals

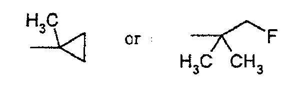

5. Compounds of general formula (Ia) 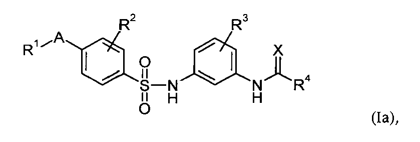 in which
R¹, R², R³, R⁴, A and X have the meanings indicated above.

6. Compounds of general formula (I) according to Claim 1, in which
A represents a 3-linked 1,2,4-oxadiazole.

7. Compounds of general formula (I) according to Claim 1, which are selected from the group consisting of the following compounds:

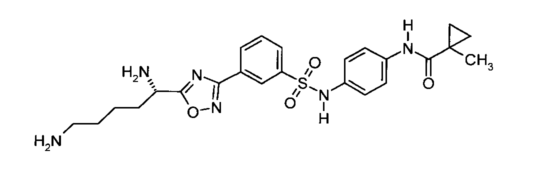

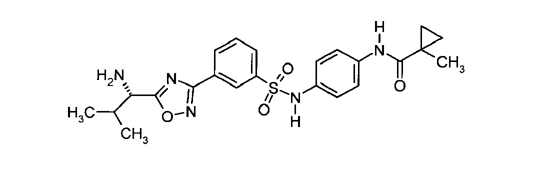

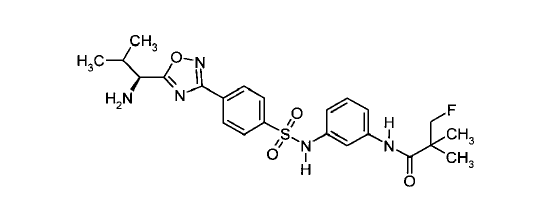

8. Method for the preparation of compounds of general formula (I) according to Claim 1, in which compounds of general formula [D-1] 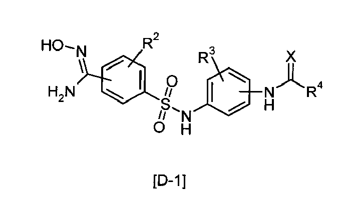 in which
X, R², R³ and R⁴ have one of the meanings indicated above, are acylated with a carboxylic acid of general formula [E-1]
R¹-COOH [E-1]
in which
R¹ has the meaning indicated above and free amino groups contained in R¹ are present protected by amino protecting groups,
in the presence of a condensing agent and of a base, and the acylated amidoxime is cyclized to the 1,2,4-oxadiazole.

9. Method for the preparation of compounds of general formula (I) according to Claim 1,
in which compounds of general formula [G-2] 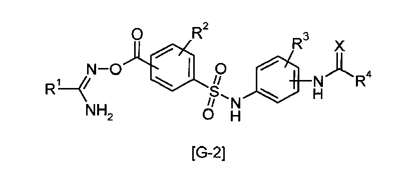 in which
R¹, R², R³, R⁴ and X have the meaning indicated above, are cyclized.

10. Compounds of general formula [D-1] 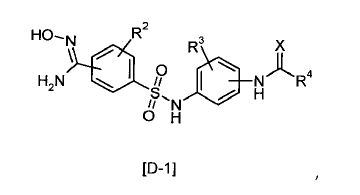 in which R², R³, R⁴ and X have the meaning indicated in Claim 1.

11. Compounds of general formula [G-2] 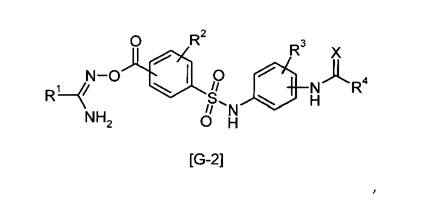 in which R¹, R², R³, R⁴ and X have the meaning indicated in Claim 1.

12. Use of compounds of general formula (I) according to any one of Claims 1 to 7 for the production of medicaments.

13. Use of compounds of general formula (I) according to Claim 12, in which the medicaments are for the control of viral diseases.

14. Use of compounds of general formula (I) according to Claim 13, in which the medicaments are for the control of cytomegalovirus infections.

15. Medicaments comprising compounds of general formula (I) according to any one of Claims 1 to 7.

## Revendications

1. Composés de la formule générale (I) 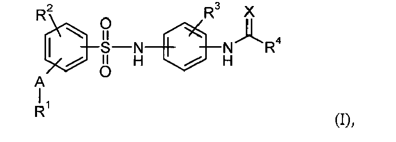 dans laquelle
R² et R³ sont identiques ou différents et représentent un hydrogène, un hydroxyle, un halogène, un nitro, un cyano, un trifluorométhyle, un trifluorométhoxyle, un alkyle en C₁ à C₆, un alcoxyle en C₁ à C₆, ou un groupe des formules 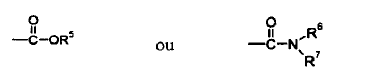 dans lesquelles
R¹, R⁶ et R⁷ sont identiques ou différents et représentent respectivement
un hydrogène ou un alkyle en C₁ à C₆, lequel peut être substitué par un ou deux substituants, choisis parmi le groupe consistant en un hydroxyle, un halogène, un cyano, un trifluorométhyle et un trifluorométhoxyle,
A représente un hétéroaryle à cinq ou six éléments, avec jusqu'à trois hé- téroatomes de la série S, N et/ou O, lié au cycle phényle avoisinant par un atome de carbone,
R1 représente le radical 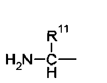
dans lequel
R¹¹ représente le groupe latéral d'un acide aminé, et dans lequel le groupe amino dans R¹ peut être substitué une ou plusieurs fois par un alkyle en C₁ à C₆, un alkylcarbonyle, un phényle, ou
R¹ représente un radical alkyle en C₁ à C₅ linéaire ou ramifié, qui peut être substitué par un ou plusieurs groupes choisis parmi un phényle, un pi- péridinyle, un pyridinyle, un thiazolyle, un thiényle, un 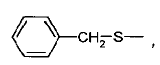 un groupe 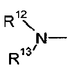 dans lequel
R¹² et R¹³ sont identiques ou différents et représentent un hydrogène, un alkyle en C₁ à C₆, un alkylcarbonyle, un groupe de protection d'amino, un phényle,
ou
R¹ représente un radical 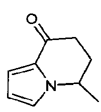 ou
R¹ représente un radical alkyle en C₁ à C₅, linéaire ou ramifié, qui est subs- titué de son côté par un groupe 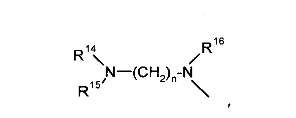
dans lequel
R¹⁴, R¹⁵, R¹⁶ sont identiques ou différents et représentent un hydrogène ou un alkyle en C₁ à C₆
et
n peut prendre les valeurs 2 ou 3,
ou
R¹ représente un pipéridinyle ou le radical 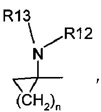 dans lequel R¹² et R¹³ on la signification spécifiée ci-dessus,
n représente un nombre de 1 à 4 et le cycle peut être substitué jusqu'à trois fois de façon identique ou différente par des halogènes, des alkyles en C₁ à C₆, des halogéno- alkyles en C₁ à C₆, des aminos, des hydroxy- les,
R⁴ représente un tertiobutyle qui est substitué le cas échéant jusqu'à trois fois, de façon identique ou différente, par un hydroxyle, un fluor ou un chlore, ou un cyclopropyle ou un cyclobutyle qui sont substitués de une à trois fois, de façon identique ou différente, par un halogène ou un alkyle en C₁ à C₆, l'alkyle en C₁ à C₆ étant substitué le cas échéant par un hy- droxyle, un fluor ou un chlore,
et dans laquelle
X représente un oxygène ou un soufre,
et dans laquelle des hétérocycles azotés peuvent aussi se présenter comme oxydes de N,
ainsi que leurs tautomères, leurs stéréo-isomères, des mélanges de leurs stéréo-isomères et leurs sels pharmacologiquement acceptables.

2. Composés de la formule générale (I) selon la revendication 1,
dans laquelle
R² et R³ sont identiques ou différents et représentent un hydrogène ou un halogène,
A représente le radical (A-I) 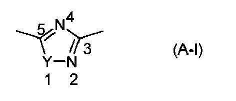
qui est relié par l'intermédiaire de l'un des atomes de carbone des positions 3 ou 5 au cycle phényle avoisinant,
et dans lequel
Y représente un oxygène,
ou
A représente le radical (A-II) 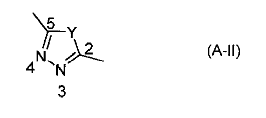
qui est relié par l'intermédiaire de l'un des atomes de carbone des positions 2 ou 5 au cycle phényle avoisinant
et dans lequel
Y représente un oxygène,
R¹ représente le radical 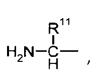
dans lequel
R¹¹ représente le groupe latéral d'un acide aminé et dans lequel le groupe amino dans R¹ peut être substitué le cas échéant une ou plusieurs fois par un alkyle en C₁ à C₆, un alkylcarbonyle, un groupe de protection d'amino, un phényle,
ou
R¹ représente un radical alkyle en C₁ à C₅ linéaire ou ramifié, qui peut être substitué par un ou plusieurs groupes choisis parmi un phényle, un pi- péridinyle, un pyridinyle, un thiazolyle, un thiényle, un phenyl, un 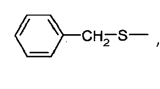 un groupe 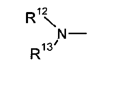 dans lequel
R¹² et R¹³ sont identiques ou différents et peuvent représenter un hydrogène, un alkyle en C₁ à C₆, un alkylcarbonyle, un groupe de protection d'amino, un phényle,
ou
R¹ représente un radical alkyle en C₁ à C₅ linéaire ou ramifié, qui est substitué de son côté par un groupe 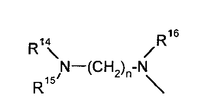
dans lequel
R¹⁴, R¹⁵, R¹⁶ sont identiques ou différents et représentent un hydrogène ou un méthyle
et
n peut prendre les valeurs 2 ou 3,
ou
R¹ représente un pipéridin-3-yle ou le radical 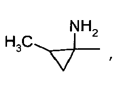
R⁴ représente un tertiobutyle qui est substitué le cas échéant jusqu'à trois fois, de façon identique ou différente, par un hydroxyle, un fluor ou un chlore, ou un cyclopropyle ou un cyclobutyle qui est substitué par un méthyle en α du groupe carbonyle ou thiocarbonyle, qui est substitué de son côté le cas échéant par un hydroxyle, un fluor ou un chlore,
et dans lequel
X représente un oxygène,
et dans lequel les hétérocycles azotés peuvent aussi se présenter comme oxydes de N,
ainsi que leurs tautomères, leurs stéréo-isomères, des mélanges de leurs stéréo-isomères et leurs sels pharmacologiquement acceptables.

3. Composés de la formule générale (I) selon la revendication 1,
dans laquelle
R² et R³ représentent un hydrogène,
A représente un des radicaux 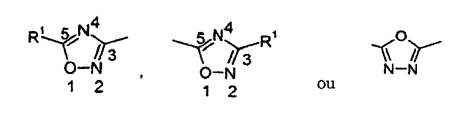
R¹ représente le radical 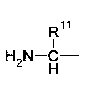
dans lequel
R¹¹ représente le groupe latéral d'un acide aminé et dans lequel le groupe amino dans R¹ peut être substitué le cas échéant une ou plusieurs fois par un méthyle, un alkylcarbonyle, un groupe de protection d'amino, un phényle,
ou
R¹ représente un radical alkyle en C₁ à C₅ linéaire ou ramifié, qui peut être substitué de son côté par un ou plusieurs groupes choisis parmi un phényle, un pipéridinyle, un pyridinyle, un thiazolyle, un thiényle, un 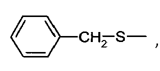 un groupe 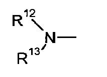
dans lequel
R¹² et R¹³ sont identiques ou différents et peuvent représenter un hy- drogène, un méthyle, un alkylcarbonyle, un groupe de protection d'amino, un phényle,
ou
R¹ représente un radical alkyle en C₁ à C₅ linéaire ou ramifié, qui est substitué de son côté par un groupe 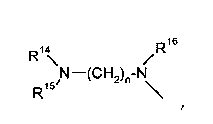
dans lequel
R¹⁴, R¹⁵, R¹⁶ sont identiques ou différents et représentent un hydrogène ou un méthyle
et
n peut prendre les valeurs 2 ou 3,
ou
R¹ représente un pipéridine-3-yle ou le radical 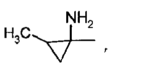
R⁴ représente un tertiobutyle qui est substitué le cas échéant jusqu'à trois fois, de façon identique ou différente, par un hydroxyle, un fluor ou un chlore, ou un cyclopropyle ou un cyclobutyle qui est substitué en α du groupe carbonyle ou thiocarbonyle par un méthyle, qui est substitué le cas échéant par un hydroxyle, un fluor ou un chlore,
et dans lequel
X représente un oxygène,
et dans lequel les hétérocycles azotés peuvent aussi se présenter comme oxydes de N,
ainsi que leurs tautomères, leurs stéréo-isomères, des mélanges de leurs stéréo-isomères et leurs sels pharmacologiquement acceptables.

4. Composés de la formule générale (I) selon la revendication 1, dans lesquels
R⁴ représente un des radicaux

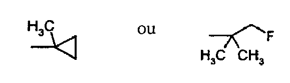

5. Composés de la formule générale (Ia) 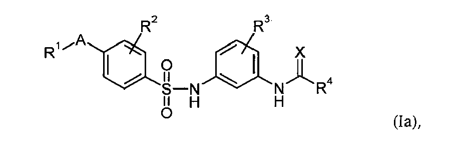 dans laquelle
R¹, R², R³, R⁴, A et X ont les significations spécifiées ci-dessus.

6. Composés de la formule générale (I) selon la revendication 1, dans lesquels
A représente un 1,2,4-oxadiazole attaché en 3.

7. Composés de la formule générale (I) selon la revendication 1, qui sont choisis parmi le groupe des composés suivants

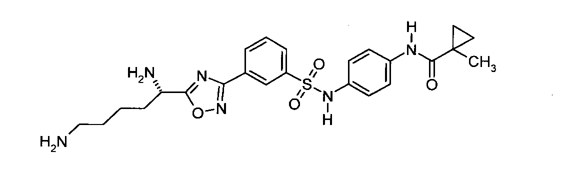

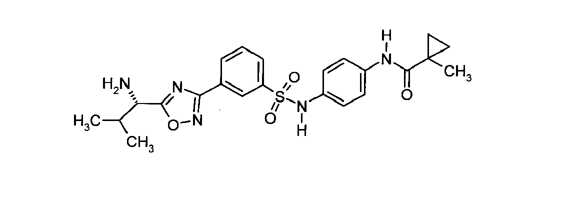

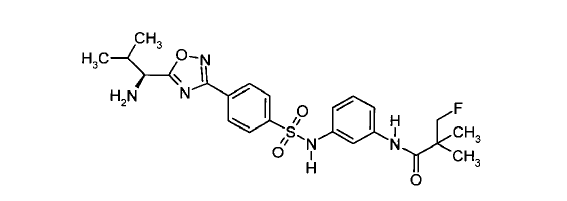

8. Procédé pour la préparation de composés de la formule générale (I) selon la revendication 1, dans lequel des composés de la formule générale [D-1] 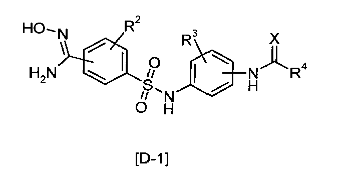 dans laquelle X, R¹, R³ et R⁴ ont une des significations spécifiées ci-dessus, sont acylés par un acide carboxylique de la formule générale [E-1]
R¹-COOH [E-1]
dans laquelle
R¹ a la signification spécifiée ci-dessus et dans laquelle les groupes amino libres contenus dans R¹ sont protégés par des groupes de protection d'amino,
en présence d'un agent de condensation et d'une base, et l'amidoxime acylé est cyclisé en 1,2,4-oxadiazole.

9. Procédé pour la préparation de composés de la formule générale (I) selon la revendication 1,
dans lequel des composés de la formule générale [G-2] 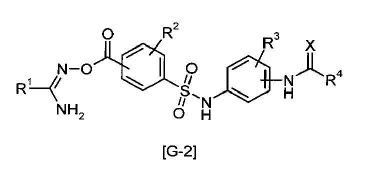 dans laquelle
R¹, R², R³, R⁴ et X ont la signification spécifiée ci-dessus, sont cyclisés.

10. Composés de la formule générale [D-1], 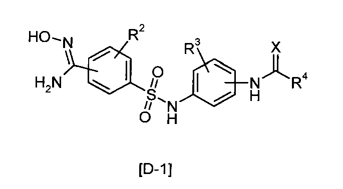 dans laquelle R², R³, R⁴ et X ont la signification spécifiée dans la revendication 1.

11. Composés de la formule générale [G-2], 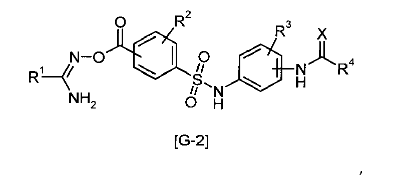 dans laquelle R¹, R², R³, R⁴ et X ont la signification spécifiée dans la revendication 1.

12. Utilisation de composés de la formule générale (I) selon l'une quelconque des revendications 1 à 7 à la préparation de produits pharmaceutiques.

13. Utilisation de composés de la formule générale (I) selon la revendication 12, dans laquelle les produits pharmaceutiques servent à la lutte contre les affections virales.

14. Utilisation de composés de la formule générale (I) selon la revendication 13, dans laquelle les produits pharmaceutiques servent à la lutte contre les infections par cytomégalovirus.

15. Produit pharmaceutique contenant des composés de la formule générale (I) selon l'une quelconque des revendications 1 à 7.
